(19) Europäisches Patentamt
European Patent Office
Office européen des brevets

(11) **EP 4 009 855 B1**

(12) **EUROPÄISCHE PATENTSCHRIFT**

(45) Veröffentlichungstag und Bekanntmachung des Hinweises auf die Patenterteilung:
**01.10.2025 Patentblatt 2025/40**

(21) Anmeldenummer: **20754219.2**

(22) Anmeldetag: **06.08.2020**

(51) Internationale Patentklassifikation (IPC):
*A61B 5/00* (2006.01)    *A61B 90/30* (2016.01)
*G01N 21/17* (2006.01)    *A61B 18/12* (2006.01)
*A61B 18/14* (2006.01)

(52) Gemeinsame Patentklassifikation (CPC):
**A61B 90/30; A61B 5/0075; A61B 5/01;**
**A61B 5/4848; A61B 18/12; A61B 18/1442;**
A61B 2018/00589; A61B 2018/00619;
A61B 2018/0063; A61B 2018/00642;
A61B 2018/00666; A61B 2018/00708;
A61B 2018/0072; A61B 2018/00732;
A61B 2018/0075;              (Forts.)

(86) Internationale Anmeldenummer:
**PCT/EP2020/072171**

(87) Internationale Veröffentlichungsnummer:
**WO 2021/023833 (11.02.2021 Gazette 2021/06)**

(54) **VORRICHTUNG ZUR BESTIMMUNG EINES ABSCHALTZEITPUNKTES EINES MEDIZINISCHEN INSTRUMENTS**

DEVICE FOR DETERMINING A SWITCH-OFF TIME OF A MEDICAL INSTRUMENT

DISPOSITIF DE DÉTERMINATION D'UN TEMPS D'ARRÊT D'UN INSTRUMENT MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.08.2019 DE 102019121375**

(43) Veröffentlichungstag der Anmeldung:
**15.06.2022 Patentblatt 2022/24**

(73) Patentinhaber: **Aesculap AG**
**78532 Tuttlingen (DE)**

(72) Erfinder:
• **HUBER, Christian**
**78570 Mühlheim (DE)**
• **ROTHWEILER, Christoph**
**78166 Donaueschingen (DE)**
• **RUSS, Detlef**
**71272 Renningen (DE)**
• **FUGGER, Oliver**
**89081 Ulm (DE)**
• **HIBST, Raimund**
**89155 Erbach (DE)**

(74) Vertreter: **Winter, Brandl - Partnerschaft mbB**
**Alois-Steinecker-Straße 22**
**85354 Freising (DE)**

(56) Entgegenhaltungen:
EP-A1- 3 011 924     US-A1- 2012 245 576
US-A1- 2016 166 309     US-A1- 2017 020 597
US-A1- 2018 345 029

(52)  Gemeinsame Patentklassifikation (CPC): (Forts.)
A61B 2018/00767; A61B 2018/00791;
A61B 2018/00875; A61B 2018/00886;
A61B 2090/309; A61B 2505/05; A61B 2560/0266;
A61B 2560/028

**Beschreibung**

**Technisches Gebiet**

Die Erfindung betrifft eine Vorrichtung und ein Verfahren zum Messen von Gewebetemperatur insbesondere von menschlichem Gewebe bei einem medizinischen Hochfrequenz-Chirurgie Instrument während eines thermischen Prozesses (HF-, Ultraschall-, Laser-Instrument etc.).

**Hintergrund der Erfindung**

Bei der Hochfrequenz-Chirurgie (im Weiteren als HF-Chirurgie bezeichnet) wird hochfrequenter Wechselstrom durch den menschlichen Körper oder ein Körperteil geleitet, um Gewebe durch die damit verursachte Erwärmung gezielt zu veröden (Koagulation) bzw. zu schneiden (Elektrotomie). Das so geschädigte Gewebe wird später vom umgebenden gesunden Gewebe resorbiert. Ein wesentlicher Vorteil gegenüber herkömmlicher Schneidetechnik mit dem Skalpell ist, dass gleichzeitig mit dem Schnitt eine Blutungsstillung durch Verschluss der betroffenen Gefäße erfolgen kann, im Sinne einer Koagulation. Für das sichere Verschließen von Gefäßen sollten sogenannte Seal&Cut Instrumentarien verwendet werden. Die benutzten Geräte werden auch als Elektroskalpell bezeichnet.

Bei den für die HF-Chirurgie (Hochfrequenz-Chirurgie) verwendeten Frequenzen verhält sich das Körpergewebe wie ein Ohm'scher Widerstand (Impedanz). Der spezifische Widerstand hängt stark von der Gewebeart ab. Der spezifische Widerstand von Muskelgewebe und stark durchblutetem Gewebe ist relativ gering. Der von Fett ist ca. um den Faktor 15 höher und der von Knochen um den Faktor 1000. Frequenz, Form und Höhe des Stroms müssen/sollten somit auf die Gewebeart, an der operiert wird, abgestimmt sein.

Derzeit wird am häufigsten die monopolare HF-Technik in der HF-Chirurgie angewendet. Dabei wird ein Pol der HF-Spannungsquelle über eine möglichst großflächige Gegenelektrode mit dem Patienten verbunden, z.B. durch Kontakte auf dem Operationstisch auf dem der Patient liegt, durch Kontaktarmbänder bzw. Kontaktfußbänder oder durch Klebeelektroden. Diese Gegenelektrode nennt man oft neutrale Elektrode bzw. Neutralelektrode. Der andere Pol wird an das chirurgische Instrument angeschlossen und dieses bildet die sogenannte aktive Elektrode bzw. Aktivelektrode. Der Strom fließt über den Weg des geringsten Widerstandes von der Aktivelektrode zur Neutralelektrode. In unmittelbarer Nähe der Aktivelektrode ist die Stromdichte am höchsten, hier findet der thermische Effekt am stärksten statt. Die Stromdichte nimmt mit dem Quadrat des Abstands ab. Die Neutrale Elektrode sollte möglichst großflächig und mit dem Körper gut verbunden sein, sodass die Stromdichte im Körper gering gehalten wird und keine Verbrennungen stattfinden. Die Haut an der Neutralelektrode wird durch die große Fläche nicht spürbar erwärmt. Bei der Anbringung der Neutralelektrode gelten strenge Sicherheitsmaßnahmen. Um keine Verbrennungen zu verursachen, sind richtige Position und guter Kontakt der neutralen Elektrode (abhängig vom Operationsgebiet) ausschlaggebend.

Bei der bipolaren HF-Technik fließt der Strom im Gegensatz zur monopolaren Technik durch einen kleinen Teil des Körpers - denjenigen, in dem die chirurgische Wirkung (Schnitt oder Koagulation) gewünscht ist. Zwei gegeneinander isolierte Elektroden (z.B. in Instrumentenbranchen aufgenommen), zwischen denen die HF-Spannung anliegt, werden direkt an die Operationsstelle geführt. Der Stromkreis wird über das dazwischen liegende Gewebe geschlossen. In dem Gewebe zwischen den Elektroden findet der thermische Effekt statt.

Koagulationsklemmen sind bekannt. Die Hochfrequenzanschlüsse sind dabei in der Regel an den Handgriffen/ dem Handgriff vorgesehen. Als Achse für das Gelenk dient häufig eine mit einem Isolierüberzug versehene Schraube, mit welcher auch die beiden Klemmschenkel mit ihren Handgriffen jeweils schwenkbar aneinander befestigt sind.

Mit einem bipolaren HF-Gefäßversiegelungs- und/oder Schneidsystem können Gefäße oder Gewebebündel allgemein oder während des Schneidens effektiv und dauerhaft versiegelt werden. Somit wird die laterale thermische Schädigung des umliegenden Gewebes begrenzt, und Gewebeanhaftungen werden auf ein Minimum reduziert.

Als Gewebe bezeichnet man in der Medizin ein organisches Material, das aus einer Gruppe gleichartig oder unterschiedlich differenzierter Zellen besteht, die eine gemeinsame Funktion oder Struktur aufweist. Zum Gewebe gehört neben den Zellen auch die extrazelluläre Matrix (EZM). Beispiele für menschliche Gewebe sind z.B. Blutgefäße.

Der menschliche Körper besteht in seiner chemischen Zusammensetzung aus ca. 56% Sauerstoff (O), 28% Kohlenstoff (C), 9% Wasserstoff (H), 2% Stickstoff (N), 1.5 % Calcium, 1% Chlor (Cl), 1% Phosphor (P), 0,25% Kalium (K), 0,2% Schwefel (S) und andere chemischen Substanzen in kleineren Anteilen (alle Angaben in Gewichtsprozent).

Die Substanzzusammensetzung des menschlichen Körpers besteht aus ca. 67% Wasser, 16% Proteine bzw. Eiweiß (z.B. Kollagene), 10% Lipide (z.B. Fett), 1% Kohlenhydrate, 1% Nucleinsäuren und 5% diverser Mineralstoffe (alle Angaben in Gewichtsprozent).

Kollagene sind eine Gruppe von bei Menschen und Tieren vorkommender Strukturproteine (ein Faserbündel bildendes "Eiweiß") hauptsächlich des Bindegewebes (genauer: der extrazellulären Matrix). Kollagene finden sich unter anderem in den weißen, unelastischen Fasern von Sehnen, Bändern, Knochen und Knorpeln. Auch Schichten der Haut (Unterhaut) bestehen aus Kollagenen. Im menschlichen Körper ist Kollagen mit über 30 % Anteil an der Gesamtmasse aller Proteine das am häufigsten vorkommende Eiweiß.

In lebenden Organismen werden Lipide hauptsächlich als Strukturkomponenten in Zellmembranen, als Energiespeicher oder als Signalmoleküle gebraucht. Oft wird der Begriff "Fett" als Synonym für Lipide gebraucht, jedoch stellen die Fette (Triglyceride) nur eine Untergruppe der Lipide dar.

Optische Hauptabsorber in Gewebe wie zum Beispiel in Blutgefäßen im NIR-Bereich sind Wasser und Kollagen. Die Blutgefäße sind meist von Fett umgeben.

Bei Wechselwirkung elektromagnetischer Strahlung mit Festkörpern, Flüssigkeiten oder Gasen treten verschiedene Effekte wie Absorption, Reflexion, Streuung oder Transmission auf. In anderen Worten, trifft die elektromagnetische Strahlung auf ein Hindernis, wird sie entweder absorbiert (verschluckt), gestreut (aus ihrer ursprünglichen Richtung abgelenkt), transmittiert (hindurchgelassen) oder reflektiert (zurückgeworfen) - man spricht auch von Remission bei der Reflexion.

Als Remission bezeichnet man in der Physik die diffuse (ungerichtete) elektromagnetische Strahlung, insbesondere von Licht, die durch die Oberfläche in ein streuendes Medium eindringt, mit diesem wechselwirkt und durch diese Oberfläche wieder austritt. Im Gegensatz zur regulären gerichteten Reflexion, die das Reflexionsgesetz erfüllt. Häufiger wird aber in beiden Fällen von Reflexion gesprochen. Man unterscheidet dann zwischen spiegelnder und diffuser Reflexion. Bei der Remission (diffusen Reflexion) wird ein Teil des Lichts absorbiert und transmittiert. Das oberflächenbezogene Maß für die Remission ist der Remissionsgrad.

Die Remissionsspektroskopie ist ein Teilgebiet der Spektroskopie, das die von einer Probe remittierte Strahlung misst. Die Remissionsspektroskopie dient vornehmlich der spektralen Untersuchung lichtundurchlässiger und unlöslicher Proben. Das gemessene Remissionsspektrum einer Probe besteht aus zwei Anteilen: 1) der regulären Reflexion, bei der die Strahlung spiegelnd von der Oberfläche reflektiert wird. Sie wird durch die Fresnelschen Gleichungen beschrieben; 2) der diffusen Remission, bei der die Strahlung isotrop in alle Richtungen aus der Probe austritt. Sie kommt dadurch zustande, dass die Strahlung in die Probe eindringt und nach teilweiser Absorption und mehrfacher Streuung an die Oberfläche zurückgelangt.

Das jeweilige Absorptionsspektrum von Wasser, Kollagen und Fett ist von zahlreichen Gruppen bereits vermessen worden. Sowohl im sichtbaren Spektralbereich (VIS) als auch im nahen Infrarotspektralbereich (NIR) sind die Werte für die Absorptionskoeffizienten verfügbar.

Die Steuerung der Regelprozesse bei der bipolaren HF-Technik findet im Stand der Technik über die Gewebeimpedanz statt, welche sich im Verlauf der Energiezufuhr, vorwiegend durch den Verlust von Wasser, ändert. Die Impedanz des Gewebes wird mittels des Ohm'schen Gesetzes anhand der gemessenen Spannungs- und Stromwerte berechnet. Aufgrund der Konfiguration eines Instruments ist die ermittelte Impedanz immer ein Durchschnittswert des gesamten Systems (Gewebe, Instrument, Kabel, Generator).

Die Qualität der Versiegelung von Blutgefäßen hängt im Wesentlichen von dem Regelprozess und dem damit verbundenen Energieeintrag in das Gewebe ab. Hierbei kann es neben der Überhitzung der Instrumente auch zur thermischen Schädigung des umliegenden Gewebes kommen. Ebenso kann ein nicht ausreichender Energieeintrag auch zum Versagen/Aufplatzen der fusionierten Stellen führen, was sich wiederum durch Blutungen bemerkbar macht. Häufig treten dies Blutungen erst Stunden nach der eigentlichen Operation auf, so dass, je nach Gefäßdurchmesser, Notoperationen erforderlich werden können, um die Blutung zu stoppen, bzw. das Gefäß sicher zu verschließen.

**Stand der Technik**

Aus dem Stand der Technik ist es daher bekannt, die Gewebetemperatur zu messen und die gemessenen Temperaturwerte in die Regelung/ Steuerung der thermischen Prozesses einfließen zu lassen. Um die Temperatur-Messergebnisse nicht durch die Elektrodentemperatur zu verfälschen, ist ein ausreichend großer Abstand, bzw. eine thermische Trennung/Isolation zwischen Gewebetemperatursensor und Elektrode(n) erforderlich. Dies ist jedoch insofern nachteilig, als dass die gemessene Gewebetemperatur nicht exakt jener Gewebetemperatur unmittelbar an der (den) Elektrode(n) entspricht.

EP 3 011 924 A1 offenbart ein Behandlungssystem, welches aufweist: ein Behandlungsinstrument, das Behandlungsenergie auf lebendes Gewebe aufbringt; eine Stromquelle, die Strom abgibt; einen Temperaturmessabschnitt zum Messen einer Temperatur des lebenden Gewebes; einen Berechnungsabschnitt, der eine Heizmenge Q berechnet, die ein zeitlicher Integralwert der Temperatur des lebenden Gewebes ist, aus der Temperatur des lebenden Gewebes und einer Zeitdauer der Aufbringung der Behandlungsenergie; einen Vergleichsabschnitt, der die Heizmenge Q und einen vorgegebenen Heizmengensollwert Qset vergleicht; und einen Anweisungsabschnitt, der eine Anweisung basierend auf einem Ergebnis des Vergleichs durch den Vergleichsabschnitt ausgibt.

US 2012/245576 A1 offenbart ein System zur Fernsteuerung eines chirurgischen Geräts, das ein Bildgebungsgerät umfasst. Das chirurgische Gerät sendet Informationen an einen Computer mit einer Software, welcher die Informationen anzeigt und/oder steuert den Betrieb des chirurgischen Geräts. Ein Display ist mit dem Computer gekoppelt, um eine grafische Benutzeroberfläche und ein einheitliches Bild anzuzeigen.

US 2016/166309 A1 offenbart ein Verfahren und ein System zum Bestimmen von Ablationsparametern zum Abtragen eines Gewebes. In einer Ausführungsform umfasst ein Verfahren das Bestimmen der Dicke eines zu abtragenden Gewebes. Das Verfahren umfasst ferner das Auswählen der Ablationstemperatur aus einem Bereich von Ablationstemperaturwerten, die für das zu abtragende Gewebe vordefiniert sind. Das Verfahren umfasst das Bestimmen der für eine effektive Läsionsbildung optimalen Gewebetemperatur über die gesamte Dicke des Gewebes, wobei die optimale Gewebetemperatur der ausgewählten Ablationstemperatur entspricht. Darüber hinaus umfasst das Verfahren das Bestimmen der optimalen Zeitdauer, die für die effektive Läsionsbildung über die gesamte Dicke des Gewebes bei der optimalen Gewebetemperatur erforderlich ist.

US 2018/345029 A1 offenbart ein Verfahren und ein System zur Erzeugung und Verteilung von Wärme in einem Zielbereich des Körpers eines Patienten zur Behandlung von Läsionen, Tumoren, Krebs, Körperschmerzen und Nervenschmerzen bereit. Die erzeugte Wärme und die Gewebetemperatur werden in Echtzeit überwacht. Das System umfasst eine Hochfrequenzantenne (RF) zum Empfangen der von einem RF-Generator erzeugten RF-Wellen. Ein RF-Absorber, der mehrere geschlossene Schleifenkreise umfasst, und ein miniaturisiertes Thermometer werden in den Körper in der Nähe des Zielgewebes implantiert. Ein Controller/Optimierer regelt eine Frequenz und einen Übertragungszeitpunkt der RF-Wellen basierend auf der gemessenen Zielgewebetemperatur. Das Thermometer, der RF-Absorber und der drahtlose Sender werden in einer Schraube platziert. Der RF-Absorber besteht aus Metall mit einer höheren RF-Absorptionsrate als die von biologischem Gewebe. Eine Ultraschallenergie wird auch zur Behandlung des Zielbereichs verwendet.

US 2017/020597 A1 offenbart Geräte, Systeme und Verfahren zur Bewertung des Erfolgs einer Behandlung, die auf Gewebe eines Patienten angewendet wird, wie etwa eine Radiofrequenzablationsbehandlung zur Neuromodulation von Nerven, die mit der Nierenarterie verbunden sind. Ein System überwacht Parameter oder Werte, die während einer Behandlung generiert werden.

## Zusammenfassung der Erfindung

Die Aufgabe der Erfindung ist es deshalb, zusätzlich oder alternativ zu der Messung der Impedanz eine möglichst exakte Messung der Temperatur des zu fusionierenden Gewebes zu ermöglichen, vorzugsweise online, um eine kontrollierte Schädigung des Gewebes unmittelbar an der (den) Elektrode(n) durchzuführen und ggf. auch eine Überhitzung der Instrumente zu verhindern. In anderen Worten ausgedrückt ist es die Aufgabe der Erfindung eine gute Koagulation zu ermöglichen, indem ein Abschaltkriterium zum Abschalten eines medizinischen Instruments bereitgestellt werden soll.

Die Aufgabe der Erfindung wird durch die Merkmale der Ansprüche 1 und 11 gelöst.

Die Erfindung betrifft ein medizinisches Instrument und ein computerlesbares Speichermedium, auf dem Anweisungen gespeichert sind, die einen Prozessor des medizinischen Instruments veranlassen, ein Verfahren zur Bestimmung eines Abschaltzeitpunkts auszuführen, umfassend die Schritte (vorzugsweise in dieser Reihenfolge):

- Messen der Dauer bei der die Temperatur des Gewebes über 85°Celsius, vorzugsweise über 95° Celsius und bevorzugt unter 110° Celsius, vorzugsweise unter 100° Celsius, liegt (Dur95),
- Berechnen der mittleren Temperatur ab dem erstmaligen Erreichen von 85°Celsius, vorzugsweise 95°Celsius und bevorzugt unter 110° Celsius, vorzugsweise unter 100° Celsius, (MeanTempabTmax),
- Messen und/oder Berechnen des Energieeintrags bis zum Erreichen der 85°Celsius, vorzugsweise der 95° und bevorzugt unter 110° Celsius, vorzugsweise unter 100° Celsius, Celsius (E2Tmax)
- Berechnen eines Parameters SP, der die vorstehenden Ergebnisse verknüpft und bei einem vorbestimmten Wert, vorzugsweise zwischen 2 und 3, besonders bevorzugt bei SP = 2.5, das medizinische Instrument abschaltet bzw. eine Abschaltung ab diesem Wert zulässt.

Ein thermischer Prozess ist vorzugsweise jeder Prozess, der durch Energieabgabe thermische Effekte im Gewebe erzeugt. Darunter zählen auch Prozesse die mittels Hochfrequenz, Ultraschall, Laser und/ oder Temperatur erfolgen. Auch zählen Prozesse darunter, die mittels Hochfrequenz-, Ultraschall-, Laser- und/ oder Temperaturinstrumenten erfolgen (z.B. mittels Thermokauter), beziehungsweise alle medizinischen Instrumente, die durch Energieabgabe thermische Effekte im Gewebe erzeugen.

Vorzugsweise weist das Verfahren ferner wenigstens einen der folgenden Schritte auf:

- Aussenden von Licht, mit einem Anregungsspektrum, vorzugsweise im VIS-/NIR-Bereich, in ein Gewebe mittels wenigstens einer Beleuchtung,
- Empfangen der Remission des Lichtes, mit einem Remissionsspektrum, aus dem Gewebe durch wenigstens einen Detektor, vorzugsweise einem Sensor,
- Umwandeln des Remissionsspektrums mittels des Detektors in ein Detektorsignal, vorzugsweise ein elektrisches Signal/Datensignal,
- Senden des Detektorsignals an eine Recheneinheit, vorzugsweise eine CPU,
- Berechnen des Remissionsspektrums aus dem Detektorsignal mittels der Recheneinheit,
- Berechnen eines Absorptionsspektrums des Gewebes durch Vergleich des Anregungsspektrum mit dem Remissionsspektrum mittels der Recheneinheit,
- Berechnen wenigstens eines Absorptionsmaximums aus dem Absorptionsspektrum mittels der Recheneinheit,
- Berechnen einer Temperatur in dem Gewebe durch Vergleich des Absorptionsmaximums mit wenigstens einer, vorzugsweise in der Recheneinheit abgespeicherten, Referenz mittels der Recheneinheit
- Berechnen eines Abschaltzeitpunktes für ein medizinisches Instrument auf Basis der Temperatur.

Vorzugsweise besteht der Schritt, des Berechnens eines Abschaltzeitpunktes für ein medizinisches Instrument auf Basis der Temperatur aus den Einzelschritten: Messen der Dauer bei der die Temperatur des Gewebes über 85°Celsius, vorzugsweise über 95° Celsius liegt und bevorzugt unter 110° Celsius, vorzugsweise unter 100° Celsius (Dur95), Berechnen der mittleren Temperatur ab dem erstmaligen Erreichen von 85°Celsius, vorzugsweise 95°Celsius und bevorzugt unter 110° Celsius, vorzugsweise unter 100° Celsius, (MeanTempabTmax), Messen und/oder Berechnen des Energieeintrags bis zum Erreichen der 85°Celsius, vorzugsweise 95° und bevorzugt unter 110° Celsius, vorzugsweise unter 100° Celsius, Celsius (E2Tmax)Berechnen eines Parameters SP, der die vorstehenden Ergebnisse verknüpft und bei einem vorbestimmten Wert, vorzugsweise zwischen 2 und 3, besonders bevorzugt bei SP = 2.5, das medizinische Instrument abschaltet bzw. eine Abschaltung ab diesem Wert zulässt. In anderen Worten ausgedrückt kann das Abschaltkriterium eine direkte Abschaltung verursachen, das bedeutet die Vorrichtung schaltet aktiv ab, und eine indirekte Abschaltung ermöglichen, das bedeutet dass eine ab diesem Wert passiv (durch einen weiteren Schritt oder durch eine Person) möglich ist / zugelassen wird (von einem Programm auf der Recheneinheit).

Vorzugsweise wird die Dauer bei der die Temperatur des Gewebes über 85°Celsius, vorzugsweise über 95° Celsius und bevorzugt unter 110° Celsius, vorzugsweise unter 100° Celsius, liegt (Dur95), dadurch gemessen, dass die Recheneinheit die Temperatur in dem Gewebe berechnet und ab dem Zeitpunkt, ab dem die Temperatur 85°Celsius, vorzugsweise 95°Celsius und bevorzugt unter 110° Celsius, vorzugsweise unter 100° Celsius, erreicht hat, die Zeit misst.

Vorzugsweise wird die mittleren Temperatur ab dem erstmaligen Erreichen von 85°Celsius, vorzugsweise 95°Celsius und bevorzugt unter 110° Celsius, vorzugsweise unter 100° Celsius, (MeanTempabTmax), von der Recheneinheit dadurch berechnet, dass online, vorzugsweise in Echtzeit, der Durchschnitt der Temperatur ab dem erstmaligen Erreichen von 85°Celsius, vorzugsweise 95 ° Celsius und bevorzugt unter 110° Celsius, vorzugsweise unter 100° Celsius, berechnet wird.

Vorzugsweise findet das Messen des Energieeintrags bis zum Erreichen der 85°Celsius, vorzugsweise 95° Celsius, (E2Tmax) dadurch statt, dass ein Leistungsmesser in das medizinische Instrument eingebracht ist. Der Leistungsmesser misst die Leistung/also die Energie, die bis zum Erreichen der 95° Celsius des Gewebes verbraucht wurde. Alternativ oder gegebenenfalls zusätzlich kann die Leistung auch anhand der Gewebeimpedanz oder der Temperaturzunahme des Gewebes über die Zeit bis zum Erreichen der 85°Celsius, vorzugsweise 95° Celsius, ermittelt bzw. berechnet werden. Der Leistungsmesser kann auch implizit in der Vorrichtung vorhanden sein durch für eine Strom- und Spannungsmessung geeignete Sensoren. In anderen Worten ausgedrückt kann die Recheneinheit die Leistung durch Daten von einer dafür vorgesehenen Strom- und Spannungsmessung berechnen.

Der Parameter SP wird berechnet, der die vorstehenden Ergebnisse verknüpft und bei einem vorbestimmten Wert, vorzugsweise zwischen 2 und 3, besonders bevorzugt bei SP = 2.5, das medizinische Instrument abschaltet, indem das Produkt aus der Dauer bei der die Temperatur eines Gewebes über 85°Celsius, vorzugsweise über 95° Celsius, liegt und der mittleren Temperatur ab dem erstmaligen Erreichen von 95°Celsius durch den Energieeintrags bis zum Erreichen von 85°Celsius, vorzugsweise 95°Celsius, dividiert wird.

Der Parameter SP bestimmt einen günstigen Zeitpunkt, für die Abschaltung eines medizinischen Instruments, vorzugsweise während eines Sealingprozesses, weiter vorzugsweise bei einem Seal&Cut Prozess. Das Produkt aus der Zeitdauer/ Dauer während der das Gewebe über 85°Celsius, vorzugsweise über 95° Celsius, gehalten wird und die durchschnittliche Temperatur während dieser Zeitspanne wird durch die Energie, die bis zum Erreichen der 85°Celsius, vorzugsweise 95°, Celsius notwendig ist, geteilt, was in dem Parameter SP resultiert. Anders ausgedrückt: Parameter SP = (Dur95*MeanTempabTmax)/E2Tmax. Ab einem Wert von SP>2,5 wird der Sealingvorgang beendet. Der Parameterwert von >2,5 stellt sicher, dass die Temperatur im Sealinggewebe nach Erreichen von 85°Celsius, vorzugsweise 95°Celsius, bzw. dem Zeitpunkt des Impedanzumschlags über einer gewisse Zeit über 85°Celsius, vorzugsweise über 95°Celsius, gehalten wird und dass die Regeltemperatur etwa bei 85°Celsius, vorzugsweise bei 95°Celsius, liegt. Dies führt dazu, dass während des Sealings ein Teil des Wassers verdampft wird, was eine Bedingung für ein erfolgreiches Sealing ist. Die Variable E2Tmax im Nenner des Quotienten ist zum einen abhängig vom Prozessverlauf der bei den unterschiedlichen Sealingprogrammen gewählt wurde und zum anderen von der Masse und Zusammensetzung des zwischen den Elektroden gefassten Gewebes. Durch die Erfassung der Größe kann man gewissermaßen die Wärmekapazität und damit auf die Masse im Gewebes in der Sealingzange schließen. Der Zähler der Gleichung lässt sich als empirische Variante des Arrhenius-Formalismus interpretieren, wonach der Denaturierungsgrad von Gewebe bei Wärmeeinwirkung vereinfacht dargestellt durch das Integral der Gewebetemperatur über die Zeit beschrieben wird.

$$\Omega(t) = A \cdot \int\limits_{0}^{t} e^{\frac{E_a}{R \cdot T(t)}} \cdot dt$$

Die Parameter Ea und A sind gewebespezifische Konstanten und R ist die allgemeine Gaskonstante. Da für ein gutes Sealingergebnis ein Teil, aber auch nicht zuviel, des Gewebewassers ausgetrieben werden muss, ist der Parameter E2Tmax im Nenner ein Korrektiv, das bei großem Gewebevolumen eine längere Einwirkdauer ergibt.

Der Parameter SP kann auf unterschiedliche Weise in den Sealingprozess integriert werden. Eine Möglichkeit dabei ist es den Parameter als Trigger für den Abschaltvorgang auf Basis des bisherigen Impedanz-Abschaltkriteriums zu integrieren. In anderen Worten ausgedrückt, wird die Beendigung des Sealings freigeben, sobald ein bestimmter Wert, bevorzugt von 2, besonders bevorzugt von 2,5, überschritten wird.

Eine weitere Möglichkeit ist, den Parameter nicht als Trigger, sondern als Abschaltkriterium zu etablieren. Sobald der Paramater den Schwellenwert von 2,5 überschritten hat, kann der Prozess beendet werden. Das Abschaltkriterium wäre dann unabhängig von der Impedanz als Kenngröße für das Abschaltkriterium.

Vorzugsweise wird die (Gewebe-)Temperatur nicht direkt gemessen, sondern über die Messung eines anderen Parameters (ungleich der Temperatur)bestimmt, der zum Einen einen unmittelbaren oder mittelbaren Rückschluss auf die aktuelle Temperatur ermöglicht (kausaler Zusammenhang zwischen der Temperatur und dem Parameter) und zum Anderen (ausschließlich) gewebespezifisch ist, d.h. nicht von der (den) Elektrode(n) beeinflusst wird. Die Gewebetemperatur wird anhand wenigstens eines Absorptionsmaximums ermittelt. Genauer gesagt wird wenigstens ein Absorptionsspektrum des Gewebes, vorzugsweise für wenigstens einen Gewebebestandteil, ermittelt, indem das Remissionsspektrum von dem Anregungsspektrum subtrahiert wird. Bevorzugte Gewebebestandteile sind Wasser, Fett und/oder Kollagen. Aus dem so erhaltenen Absorptionsspektrum kann ermittelt werden an welcher Stelle sich wenigstens ein Absorptionsmaximum des Absorptionsspektrums des Gewebes, vorzugsweise wenigstens eines Gewebebestandteils, befindet. Das Absorptionsmaximum, vorzugsweise die Frequenz, Wellenlänge oder Position des Absorptionsmaximums, wird verglichen mit wenigstens einer auf der Recheneinheit, vorzugsweise auf dem Speichermedium, gespeicherten Referenz. Die wenigstens eine gespeicherte Referenz kann dann aus einer Tabelle oder mittels einer Referenzmessung ermittelt werden, so dass festgestellt werden kann, dass bei einer bestimmten Position/Wellenlänge/Frequenz des Absorptionsmaximums in dem Gewebe eine bestimmte Temperatur herrscht. Sollte diese Position/Wellenlänge/-

Frequenz des Absorptionsmaximums nicht in einer Tabelle gespeichert sein, kann mittels einer Verschiebung der berechneten Position/Wellenlänge/Frequenz des Absorptionsmaximums von einer hinterlegten Position/-Wellenlänge/Frequenz des Absorptionsmaximums auf der Recheneinheit die Temperatur in dem Gewebe berechnet werden. Statt des Absorptionsmaximums kann auch jede andere Position/Wellenlänge/Frequenz aus dem Absorptionsspektrum verwendet werden, die einen signifikanten Wiedererkennungswert aufweist (z.B. Maxima oder Minima).

Die Gewebeanteile des Gewebes weisen eine typische Absorptionskennlinie auf. So hat Wasser ein Absorptionsmaxiumum bei ca. 1470nm bei Raumtemperatur, Kollagen dagegen hat ein Absorptionsmaximum bei ca. 1500nm bei Raumtemperatur und Fett hat je ein Absorptionsmaximum bei 1210nm und bei ca. 1400nm bei Raumtemperatur. Vorzugsweise ist das Absorptionsmaximum von Wasser bei 1470 nm +/- 20 nm, besonders bevorzugt bei 1470 nm +/- 10 nm, besonders bevorzugt bei 1470 nm +/- 5 nm. Vorzugsweise ist das Absorptionsmaximum von Kollagen bei 1500 nm +/- 20 nm, besonders bevorzugt bei 1500 nm +/- 10 nm, besonders bevorzugt bei 1500 nm +/- 5 nm. Vorzugsweise ist das Absorptionsmaximum von Fett bei 1210 und bei 1400 nm +/-20 nm, besonders bevorzugt bei 1210 und bei 1400 nm +/- 10 nm, besonders bevorzugt bei 1210 und bei 1400 nm +/- 5 nm.

Vorzugsweise weist das Verfahren ferner den Schritt auf:

- Speichern wenigstens einer Referenz in Form eines Absorptionsmaximums bei einer bestimmten Temperatur in der Recheneinheit, vorzugsweise einem Speichermedium in der Recheneinheit, vorzugsweise für Wasser und/oder Fett und/oder Kollagen.

Vorzugsweise kann mittels der Recheneinheit anhand des charakteristischen Absorptionsspektrums von Wasser als Referenz ermittelt werden, welche Temperatur in dem Gewebe herrscht. Auf der Recheneinheit bzw. dem Speichermedium ist gespeichert, dass Wasser bei einer bestimmten Temperatur ein bestimmtes Absorptionsmaximum (z.B. bei Raumtemperatur 1470nm) hat. Durch vergleichen der Verschiebung der Absorptionsmaxima von einem vorgespeicherten Wert und/oder durch vergleichen mit einer Vielzahl vorgegebener korrespondierender Werte in einer gespeicherten Tabelle kann ermittelt werden, bei welcher Wellenlänge des Absorptionsmaximums welche Temperatur in dem Wasser des Gewebes herrscht. Das charakteristische Absorptionsspektrum von Wasser kann am einfachsten ermittelt werden, da die Gewebebestandteile im Körper bekannt sind und Wasser mit ca. 67% am stärksten im Gewebe vorhanden ist. Aufgrund des gemessenen Absorptionsspektrums kann die Verschiebung des spektralen Absorptionsmaximums von Wasser berechnet/ermittelt werden. Anhand dieser Verschiebung des Absorptionsmaximums, die ca. 0,5nm/K beträgt, kann die Temperatur bestimmt werden. Das vorstehende ist analog anwendbar auf Fett und/oder Kollagen und/oder anderen Bestandteilen des Gewebes.

Die vorstehenden Schritte zur Messung des Absorptionsspektrums können neben Wasser auch für Fett, Kollagen oder anderen Gewebebestandteilen analog angewendet werden. Somit können aus einem Absorptionsspektrum, das von einem Detektor erfasst und von einer Recheneinheit ermittelt wird, die einzelnen Absorptionsspektren von Wasser, Fett und Kollagen in Gewebe ermittelt werden.

Vorzugsweise weist das Verfahren ferner den Schritt auf:

- Aufbringen der Beleuchtung und des Detektors auf das Gewebe. Vorteilhafterweise sind der Detektor und die Beleuchtung somit in direktem Kontakt mit dem Gewebe.

Vorzugsweise weist das Verfahren ferner den Schritt auf:

- Steuern und/oder Regeln und/oder Abschalten einer Vorrichtung, vorzugsweise eines medizinischen Instruments, mittels der Recheneinheit auf Basis der berechneten Temperatur und/oder Gewebeimpedanz.

Vorzugsweise findet das Steuern und/oder Regeln und/oder Abschalten bei Erreichen einer vorbestimmten Temperatur, vorzugsweise bei einer Temperatur die größer 85°Celsius, vorzugsweise größer 95° Celsius, und kleiner 110°Celsius statt, vorzugsweise kleiner 100° Celsius. Die Koagulation von Gewebe erreicht das beste Ergebnis bei einer Temperatur, vorzugsweise konstanten Temperatur, über 85°Celsius, vorzugsweise über 95° Celsius, und weiter vorzugsweise bei einer Temperatur von unter 110° Celsius, vorzugsweise 100° Celsius.

Vorzugsweise finden alle Schritte online/ in Echtzeit statt. Das bedeutet, dass das Steuern und/oder das Regeln und/oder das Abschalten des medizinischen Instruments online, vorzugsweise in Echtzeit stattfindet. In anderen Worten ausgedrückt wird das Absorptionsspektrum des Gewebes online, vorzugsweise in Echtzeit gemessen, wodurch die Temperatur in dem Gewebe online, also in Echtzeit berechnet werden kann. Die Temperatur fließt dann vorzugsweise online, vorzugsweise in Echtzeit, in die Steuerung/ Regelung wenigstens einer Elektrode/ Sonotrode/ Laserquelle des medizinischen Instruments, vorzugsweise der Cut&Seal-Vorrichtung, mit ein.

Vorzugsweise wird das Verfahren zur Temperaturmessung während eines Sealingvorgangs durchgeführt, besonders bevorzugt im Gewebe im medizinischen Instrument.

Vorzugsweise sind die Detektoren vorgesehen und angepasst Remission, vorzugsweise die Remissionspektren, im NIR-Bereich von 1000nm bis 1700nm, besonders bevorzugt im Bereich von 1400nm bis 1600nm zu erfassen.

Vorzugsweise sind die wenigstens eine Beleuchtung und der wenigstens eine Detektor beabstandet, vorzugsweise in einem medizinischen Instrument.

Vorzugsweise findet das Verfahren zur Messung einer Gewebetemperatur Anwendung in einem medizinischen Instrument.

Vorzugsweise weist eine Temperaturmessvorrichtung ein Speichermedium auf, auf dem wenigstens einer der folgenden Schritte gespeichert ist (bei einer Mehrzahl vorzugsweise in dieser Reihenfolge):

- Speichern wenigstens einer Referenz in Form eines Absorptionsmaximums bei einer bestimmten Temperatur in der Recheneinheit, vorzugsweise einem Speichermedium in der Recheneinheit, vorzugsweise für Wasser und/oder Fett und/oder Kollagen.

- Aufbringen der Beleuchtung und des Detektors auf das Gewebe. Vorteilhafterweise sind der Detektor und die Beleuchtung somit in direktem Kontakt mit dem Gewebe.

- Aussenden von Licht, mit einem Beleuchtungsspektrum, vorzugsweise im VIS-/NIR-Bereich, in ein Gewebe mittels wenigstens einer Beleuchtung,

- Empfangen der Remission des Lichtes, mit einem Remissionsspektrum, aus dem Gewebe durch wenigstens einen Detektor, vorzugsweise einem Sensor,

- Umwandeln des Remissionsspektrums mittels des Detektors in ein Detektorsignal, vorzugsweise ein elektrisches Signal/Datensignal,

- Senden des Detektorsignals an eine Recheneinheit, vorzugsweise eine CPU,

- Berechnen des Remissionsspektrums aus dem Detektorsignal mittels der Recheneinheit,

- Berechnen eines Absorptionsspektrums des Gewebes durch Vergleich des Beleuchtungsspektrum mit dem Remissionsspektrum mittels der Recheneinheit,

- Berechnen wenigstens eines Absorptionsmaximums aus dem Absorptionsspektrum mittels der Recheneinheit,

- Berechnen einer Temperatur in dem Gewebe durch Vergleich des Absorptionsmaximums mit wenigstens einer, vorzugsweise in der Recheneinheit abgespeicherten, Referenz mittels der Recheneinheit,

- Steuern und/oder Regeln und/oder Abschalten einer Vorrichtung, vorzugsweise eines medizinischen Instruments, mittels der Recheneinheit auf Basis der berechneten Temperatur und/oder Gewebeimpedanz.

In anderen Worten ausgedrückt werden in der Temperaturmessung während eines Sealingvorgangs online Remissionspektren im NIR-Bereich von 1000nm bis 1700nm von einem Detektor erfasst. Die aus den aufgenommenen Spektren ableitbare Verschiebung der Lage der Absorptionsmaxima kann genutzt werden, um auf die Temperatur des im Instrument gefassten Gewebes, mit für die Anwendung ausreichender Genauigkeit, zu schließen, Mit zunehmender Temperatur verschiebt sich die Lage des Absorptionspeaks hin zu kürzeren Wellenlängen. Die Verschiebung ist dabei etwa 0,5nm/K. Kühlt das Gewebe weiter ab, verschiebt sich der Absorptionspeak wieder in Richtung längerer Wellenlängen. Da der Hauptabsorber im zu sealenden Gewebe im Wellenlängenbereich um ca. 1470 nm Wasser ist, spiegelt die so bestimmte Temperatur die Temperatur im Wasseranteil des Gewebes wieder. Der besondere Vorteil dieses Temperaturmessverfahrens ist, dass damit die tatsächliche Temperatur im Gewebe gemessen werden kann, da die NIR-Strahlung aufgrund der Streuung die gesamte Dicke der Gewebeschicht passieren kann. Im Gegensatz dazu wird bei der Messung der Temperatur während des Sealings mit einem Thermoelement lediglich die Temperatur der Kontaktfläche gemessen. Die Temperatur und die Wärmekapazität der Elektroden stellt für die Bestimmung der Gewebetemperatur bei dieser Methode eine Störgröße dar. Diese führt zu Latenzzeiten und Verfälschungen der wahren Gewebetemperatur. Diese Methode spiegelt somit nicht die Gewebetemperatur wider, sondern repräsentiert die Temperatur der Umgebung, mit der das Thermoelement in Kontakt ist. Mit der optischen Temperaturbestimmung ist es möglich wichtige Parameter für die Steuerung des Sealingprozesses zu gewinnen. Weiter kann die ermittelte Temperatur als Abschalt-/Regelungs-/Steuer-Kriterium/ Prozessparameter verwendet werden bzw. zur Prozessregelung/-Prozesssteuerung.

Es hat sich gezeigt, dass Licht vorzugsweise bestimmter Wellenlänge (z.B. Weißlicht im VIS-NIR-Bereich) vom Körpergewebe remittiert wird, wobei sich das Spektrum des vom Körpergewebe remittierten Lichts temperaturabhängig ändert. Es ist also möglich, eine Beleuchtung/Beleuchtungsausgang zur Bestrahlung von Körpergewebe sowie einen Detektor/Detektoreingang zur Erfassung von vom Körpergewebe remittiertem Licht unmittelbar an die Elektrode(n) heranzuführen und so die Gewebetemperatur in unmittelbarer Nähe zu (zwischen) der (den) Elektrode(n) über den Umweg des erfassten remittierten Lichts und dessen spektraler Verteilung zu bestimmen.

In der bevorzugten Ausführungsform hat ein medizinisches Instrument (der HF-Bauart) demzufolge

- wenigstens eine Instrumentenbranche, die wenigstens eine bestrombare Elektrode zum Versiegeln und/oder Schneiden von Gewebe bildet oder in oder an der wenigstens einen bestrombaren Elektrode zum Versiegeln und/oder Schneiden von Gewebe angeordnet ist, wobei die Bestromung der Elektrode durch eine Recheneinheit steuerbar und/oder regelbar ist, und

- wenigstens eine Temperaturmessvorrichtung, mit wenigstens einer Beleuchtung und wenigstens einem Licht-Detektor, die jeweils in oder an der zumindest einen Instrumentenbranche oder in Gegenüberlage in/an zwei Instrumentenbranchen (wechselweise) ausgebildet oder angeordnet ist/sind und die in elektrischer Verbindung mit der Recheneinheit stehen.

Vorzugsweise ist das medizinische Instrument ein chirurgisches Instrument, ein monopolares Instrument, ein bipolares Instrument, ein elektrochirurgisches Instrument, eine chirurgische Klammer, eine chirurgische Klemme, eine chirurgische Zange, eine chirurgische Schere, ein Skalpell und/oder dergleichen. Besonders bevorzugt ist das medizinische Instrument ein Seal&Cut-Instrument, das vorgesehen und angepasst ist, Gewebe mittels HF-Technologie zu schneiden und gleichzeitig zu versiegeln. Monopolare Instrumente haben den Vorteil, dass dadurch, dass sie einschalig ausgeformt sind (nur eine einzige Instrumentenbranche), eine kompakte Bauform ermöglicht wird und somit geringere Kosten bei deren Herstellung. Bipolare Instrumente (zwei gegenüberliegende Instrumentenbranchen) haben den Vorteil, dass eine aufgelöste Analyse besser umsetzbar ist und dass sie variabler in der Umsetzung der Duplizierung sind.

Vorzugsweise ist die mindestens eine Instrumentenbranche als der Teil /das Ende eines medizinischen Instruments zu verstehen, der/das mit dem Gewebe in Kontakt bringbar ist. Weiter bevorzugt ist die mindestens eine Instrumentenbranche eine Maulteilbranche. Die zumindest eine Instrumentenbranche kann als Elektrode zum Versiegeln von Gewebe ausgebildet sein, vorzugsweise ist die Instrumentenbranche dabei einstückig/ aus einem einzigen Teil aus einem leitfähigen Metall oder Graphit. Alternativ kann die Elektrode in und/oder an und/oder auf der Instrumentenbranche ausgebildet/ angeordnet/ eingebettet sein, vorzugsweise ist in diesem Fall die Instrumentenbranche dabei aus einem Isolator bzw. elektrisch isolierenden Material.

Vorzugsweise weist das medizinische Instrument zwei sich gegenüberliegende Instrumentenbranchen auf, die vorzugsweise gegeneinander bewegbaren/ schwenkbar sind, an deren Enden einander zugewandten Seiten/ Backen/ Bereiche/ Instrumentenbranchenenden angeordnet/ausgebildet sind, die mit dem Gewebe in Kontakt bringbar sind. Die Instrumentenbranchen können selbst als Elektroden zum Versiegeln von Gewebe ausgebildet sein, vorzugsweise sind die Instrumentenbranchen dabei aus einem leitfähigen Metall oder Graphit und gegeneinander isoliert. Die Elektroden können aber auch in und/oder an und/oder auf den Instrumentenbranchen ausgebildet/ angeordnet/ eingebettet sein, vorzugsweise sind die Instrumentenbranchen dabei aus einem Isolator bzw. elektrisch isolierenden Material oder sind aus Metall und gegen die Elektroden isoliert

Vorzugsweise ist mindestens eine Elektrode durch die Recheneinheit steuerbar und/oder regelbar. Genauer gesagt ist die Stromstärke, die Spannung, die Phase und/oder die Frequenz des elektrischen Stroms, der/die an der Elektrode anliegt, steuerbar oder regelbar.

Vorzugsweise ist die Temperaturmessvorrichtung eine optische Temperaturmessvorrichtung/ ein Thermometer mit einem optischen Sender in Form einer Beleuchtung und einem optischen Empfänger in Form eines Licht-Detektors.

Vorzugsweise ist unter Beleuchtung wenigstens eine Lichtquelle/ Anregungslichtquelle und alternativ zusätzlich andere optische Bauteile zu verstehen, wie zum Beispiel ein Lichttunnel, der Lichtwellenleiter/ Spiegel/ Linsen/ reflektierende Innenwände/ streuende Medien und dergleichen aufweist. Weiter bevorzugt ist unter Lichtquelle eine Weißlichtquelle/ eine LED (im VIS- und/oder IR- und/oder UV-Bereich), eine Deuteriumlampe (UV-Bereich) und/oder eine Halogenlampe (VIS-Bereich) zu verstehen. In anderen Worten ausgedrückt kann das Licht an/in/auf der Instrumentenbranche an dem Einstrahlort/ an der wenigstens einen Eintrittsöffnung direkt mittels einer Lichtquelle erzeugt werden oder indem das Licht von einer Lichtquelle mittels Lichtwellenleiter/ Spiegel/ Linsen/ Lichttunnel/ streuende Medien und dergleichen an einen Einstrahlort/ eine Lichteinlassöffnung/ eine Lichteintrittsöffnung der Kontaktfläche der Instrumentenbranche, die vorgesehen und angepasst ist mit dem Gewebe in Kontakt zu kommen, geführt wird. Weiter bevorzugt erfolgt die Einstrahlung des Lichts der Beleuchtung in einem bestimmten Winkel relativ zu der Gewebekontaktfläche der entsprechenden Instrumentenbranche bzw. Elektrode, das heißt die Beleuchtung weist in/an/auf der Instrumentenbranche eine gewinkelte/ schräggestellte Austrittsöffnung und/oder Lichtstrahlung auf. In wieder anderen Worten ist die Lichtquelle selbst schräg/angewinkelt auf/an/in der Instrumentenbranche angeordnet oder weist eine schräge/ gewinkelte Oberfläche bezüglich der Gewebekontaktfläche bzw. Lichtaustrittsfläche auf. Alternativ kann ein optisches Element wie zum Beispiel ein Spiegel und/oder ein Lichtwellenleiter schräg auf/an/in der Kontaktoberfläche (der Fläche die vorgesehen und angepasst ist mit Gewebe in Kontakt zu kommen) der Instrumentenbranche angeordnet sein und das Licht von der Lichtquelle zu dem Einstrahlort bzw. der Kontaktfläche führen.

Eine Weißlichtquelle, also eine Lichtquelle, die elektromagnetische Strahlung über den ganzen VIS-Bereich aussendet, hat den Vorteil, dass mehr Information aus dem zu beleuchtenden Gewebe gewonnen werden kann, wodurch eine Gewebeerkennung und/oder eine multivariate Datenanalyse möglich sind. Des Weiteren besteht die Möglichkeit, eine Vielzahl verschiedener Messungen durchzuführen. Beispielsweise kann auf der Instrumentenbranche wenigstens eine Beleuchtung mit einer Weißlichtquelle und wenigstens ein Detektor angeordnet sein der vorgesehen und angepasst ist, spektrale Bereiche zu messen, vorzugsweise mit verschiedenen Sensoren (Si-, InGaAs-Sensoren usw.).

Eine Lichtquelle mit geringer spektraler Bandbreite hat den Vorteil, dass die Umsetzung einfach erfolgt, dass eine solche Lichtquelle kostengünstig ist, dass eine hohe zeitliche Abtastung mit einer solchen Lichtquelle erreicht werden kann und dass Abstände von mehr als 2 mm voneinander und/oder zu einem Detektor möglich sind, da eine höhere Intensität auf einen bestimmten Spektralbereich möglich ist.

Vorzugsweise ist unter Detektor bzw. Licht-Detektor wenigstens ein Sensor/ eine Photodiode und/oder ein Photomultiplier (PMT) und ggf. andere optische Bauteile zu verstehen, wie zum Beispiel ein Lichttunnel, der Lichtwellenleiter/ Spiegel/ Linsen/ / reflektierende Innenwände/ streuende Medien und dergleichen aufweisen kann. In anderen Worten ausgedrückt kann das Licht von dem in/an/auf der Instrumentenbranche eingebauten Detektor/ Detektorteil an dem Remissionsort direkt mittels eines dort angeordneten Sensors des Detektors oder dergleichen an/in/auf der Instrumentenbranche gemessen werden oder über einen Lichttunnel, der Lichtwellenleiter/ Spiegel/ Linsen/ / reflektierende Innenwände/ streuende Medien und dergleichen aufweisen kann und Licht von der Kontaktfläche/einer Lichteintrittsöffnung der Instrumentenbranche zu einem entfernt von der Kontaktfläche der Instrumentenbranche oder gar entfernt von der Instrumentenbranche angeordneten Sensor oder dergleichen geführt werden. Weiter bevorzugt erfolgt die Einstrahlung des Lichts ausgehend von der Beleuchtung in einem bestimmten Winkel (0° < Winkel ≤ 90°) relativ zu der Gewebekontaktfläche der entsprechenden Instrumentenbranche bzw. Elektrode. Weiter bevorzugt weist der Detektor in/an/auf der Instrumentenbranche eine zur Kontaktfläche ebenfalls gewinkelte/ schräggestellte Eintrittsöffnung auf. In wieder anderen Worten ist der Detektor selbst schräg/angewinkelt auf/-an/in der Instrumentenbranche angeordnet oder weist eine schräge/ gewinkelte Oberfläche bezüglich der Gewebekontaktfläche auf. Alternativ kann ein optisches Element wie zum Beispiel ein Spiegel und/oder ein Lichtwellenleiter schräg auf/an/in der Kontaktoberfläche (der Fläche, die vorgesehen und angepasst ist mit Gewebe in Kontakt zu kommen) der Instrumentenbranche angeordnet sein und Remissionslicht zu einem entfernten Sensor oder dergleichen führen. Das nach der Einstrahlung vom Körpergewebe remittierte Licht wird vorzugsweise in mindestens zwei Kanäle spektral aufgelöst (mittels Spektrometer, Prismen oder unterschiedlichen Filtern) und dann von den mindestens zwei Sensoren oder dergleichen erfasst, die in Abhängigkeit hiervon mindestens zwei Signale zu der Recheneinheit/CPU senden, welche die mindestens zwei Signal in einen Temperaturwert transformiert.

Die Elektrode zum Versiegeln von Gewebe ist vorzugsweise aus Metall, leitfähiger Keramik, metallisierter Keramik, Graphit oder metallisiertem Graphit. Die Elektrode ist weiter vorzugsweise mit einer Oberfläche ausgebildet, die vorgesehen und angepasst ist, elektromagnetische Strahlung zu reflektieren.

Die Recheneinheit weist vorzugsweise einen Prozessor und ein Speichermedium auf. Das Speichermedium ist vorgesehen und angepasst, Schritte zur Ausführung der Messung der Temperatur und/oder der Steuerung und/oder Regelung des Stroms der Elektrode zu speichern.

Die Recheneinheit steuert die Beleuchtung/-Lichtquelle der Beleuchtung (Dauer, Intensität, Wellenlänge usw.) mittels eines ersten elektrischen Signals und der Detektor erfasst das (ausschließlich) vom Körpergewebe gestreute/ reflektierte Licht bzw. die Remission unmittelbar an dem zu messenden/ behandelnden Gewebe (zwischen den Instrumentenbranchen) und sendet die ermittelten Daten als ein zweites elektrisches Signal an die Recheneinheit. Die Recheneinheit berechnet nun mittels eines Algorithmus auf dem Speichermedium, die aus dem jeweiligen zweiten elektrischen Signal ableitbare Temperatur des Gewebes. Auf Basis der so berechneten Temperatur des Gewebes wird online/ in Echtzeit berechnet welche Stromstärke, welche Spannung und/ oder welche Frequenz der elektrische Strom haben soll, welcher an die wenigstens eine Elektrode angelegt wird.

Zusätzlich kann in einer Ausführungsform auch der Widerstand des Gewebes (Gewebeimpedanz) von der Recheneinheit ermittelt werden und in die Berechnung mit einfließen. In anderen Worten kann die Gewebeimpedanz des Gewebes an/ zwischen den Elektroden/ Sonotroden ermittelt werden, sodass die Stromstärke, Spannung und/oder Frequenz des an die Elektrode(n), bzw. des US-Wandlers angelegten elektrischen Stroms in Erwiderung auf die ermittelte Gewebeimpedanz und (in Kombination mit) dem zweiten Signal der (optischen) Temperaturmessvorrichtung von der Recheneinheit gesteuert oder geregelt werden kann.

Vorzugsweise steht die Recheneinheit mit der erfindungsgemäßen (optischen) Temperaturmessvorrichtung so in Verbindung, dass die Stromstärke, die Spannung und/ oder die Frequenz des elektrischen Stroms, der an der wenigstens einen Elektrode anliegt in Antwort auf die von der Recheneinheit/CPU berechnete Temperatur änderbar ist, vorzugsweise automatisch und/oder durch einen vorgegebenen Algorithmus.

Vorzugsweise entspricht das zweite elektrische Signal von dem Detektor einem Lichtspektrum, das die Wellenlänge und die Intensität des am Detektor erfassten Lichts darstellt. Aufgrund dieses Spektrum wird die Verschiebung des spektrales Absorptionsmaximums von Wasser berechnet/ermittelt. Anhand dieser Verschiebung des Absorptionsmaximums, die ca. 0,5nm/K beträgt, kann die Temperatur bestimmt werden. Da das Absorptionsspektrum von Wasser charakteristisch ist, kann die Verschiebung auch ohne Referenzmessung und/oder mit Referenzmessung ermittelt werden.

Vorzugsweise ist die Recheneinheit so konfiguriert, dass sie wenigstens einen der folgenden Schritte aufweist oder es ist auf einem Speichermedium in der Recheneinheit wenigstens einer der folgenden Schritte gespeichert (vorzugsweise in der folgenden Reihenfolge):

- Ansteuern der Beleuchtung durch die Recheneinheit mit einem ersten elektrischen Signal, vorzugsweise mit einem elektrischen Strom mit bestimmter Stromstärke und/oder einer bestimmten Spannung und/oder einer bestimmten Frequenz,
- Aussenden einer elektromagnetischen Strahlung der der Beleuchtung (vorzugsweise Weißlicht) in das Gewebe, in einem bestimmten Bereich in unmittelbarer Nähe zu einer Elektrode oder zwischen zwei sich gegenüberliegenden Elektroden,
- Messen (mittels des Detektors) der Remission/ der diffusen Reflexion der elektromagnetischen Strahlung ausgehend vom Körpergewebe,
- Senden der Messergebnisse von dem Detektor an die Recheneinheit mittels eines zweiten elektrischen Signale,
- Transformieren des zweiten elektrischen Signals in einen Gewebetemperaturwert,
- Vorzugsweise Ermitteln der Gewebeimpedanz, vorzugsweise zwischen zwei Elektroden,
- Verarbeiten des Gewebetemperaturwerts und vorzugsweise der ermittelten Gewebeimpedanz mittels der Recheneinheit, vorzugsweise mittels eines vorprogrammierten Algorithmus auf dem Speichermedium, zur Bestimmung einer neuen Stromstärke, Spannung und/oder Frequenz für den an die Elektrode(n) angelegten elektrischen Strom zur Erreichung oder Annäherung einer Temperatur des Gewebes von über 85°Celsius, vorzugsweise über 95° Celsius, und bevorzugt gleichzeitig von unter 110°Celsius, vorzugsweise von unter 100°Celsius.;
- Kontinuierliche Berechnung des Parameters SP aus den kontinuierlich bestimmten Werten Dur95, MeanTempabTmax und E2Tmax;
- Bereitstellung des Abschalttriggers.

In einer Ausführungsform kann der Lichttunnel, der mit der Lichtquelle in Verbindung steht, an wenigstens einem Ende von wenigstens einer Lichtquelle gespeist werden und das wenigstens eine andere Ende in der Instrumentenbranche enden. In anderen Worten ausgedrückt kann Licht von wenigstens einer Lichtquelle über einen Lichtwellenleiter oder dergleichen an wenigstens einen Ausgang geleitet werden, der sich an/auf/in der Instrumentenbranche befindet. Alternativ kann sich wenigstens eine Lichtquelle, z.B. die LED, direkt auf/-an/in der Instrumentenbranche befinden/angeordnet sein.

In einer Ausführungsform kann der Lichttunnel, der mit dem Detektor in Verbindung steht, an wenigstens einem Ende wenigstens einen Sensor aufweisen und an dem wenigstens einen anderen Ende in der Instrumentenbranche enden. In anderen Worten ausgedrückt kann Licht/Remission von wenigstens einem Eingang, der sich auf/in der Instrumentenbranche befindet, über einen reflektierenden Lichtkanal/ einen Lichtwellenleiter oder dergleichen an wenigstens einen Sensor/ eine Photodiode/ einen Photomultiplier oder dergleichen geleitet werden. Alternativ kann sich wenigstens ein Sensor/ Photodiode/ Photomultiplier auf/an/in der Instrumentenbranche befinden/angeordnet sein.

Vorzugsweise können sich die Beleuchtung und der Detektor ein Ende eines Lichttunnels teilen. In anderen Worten ausgedrückt können sich der Strahlengang der Lichtquelle und der Strahlengang des Sensors/ Photodiode/ Photomultipliers einen Lichttunnel teilen, so dass beides über eine einzige optische Öffnung, die gleichzeitig den Eingang und den Ausgang des Lichts auf/an/in der Instrumentenbranche bildet, mit dem Körpergewebe in optischem Kontakt ist.

Vorzugsweise sind eine Mehrzahl von Detektoren und eine Mehrzahl von Beleuchtungen auf wenigstens einer Instrumentenbranche angeordnet. Dabei können die Detektoren bzw. Beleuchtungen jeweils auf einer Instrumentenbranche in einem vorgegebenen Muster angeordnet sein. Das Muster ist vorzugsweise linienförmig. Alternativ kann/können wenigstens ein Detektor und/oder eine Beleuchtung auf einer ersten Instrumentenbranche angeordnet sein und wenigstens ein Detektor und/oder eine Beleuchtung auf einer zweiten Instrumentenbranche angeordnet sein, vorzugsweise auf einander zugewandten Seiten von gegenüberliegenden Instrumentenbranchen. In anderen Worten ausgedrückt kann in dieser Ausführungsform für bipolare Instrumente das Licht von einer Beleuchtungseinrichtung in das Gewebe eingebracht werden und auf einer gegenüberliegenden Seite kann ein Detektor das vom Gewebe remittierte Licht messen.

Vorzugsweise beträgt die Distanz zwischen der wenigstens einen Beleuchtung und dem wenigsten einen Detektor zwischen 0 und 5 mm, besonders bevorzugt zwischen 0 und 1 mm, da dort die Intensität der Remission sehr hoch ist.

Vorzugsweise weist die wenigstens eine Instrumentenbranche pro Beleuchtung mehrere Detektoren auf, besonders bevorzugt sind die Detektoren in gleichen und/oder unterschiedlichen Abständen zu der Beleuchtung angeordnet. In anderen Worten ausgedrückt kann der Abstand von einer Beleuchtung zu einem zweiten Detektor größer sein als der Abstand zu einem ersten Detektor.

Vorzugsweise weist die Beleuchtung eine diskrete Lichtquelle auf, vorzugsweise mit einer definierten Bandbreite, besonders bevorzugt mit einer Bandbreite kleiner 100 nm.

Vorzugsweise ist die (optische) Temperaturmessvorrichtung auf einer Ebene der Instrumentenbranche angeordnet, die tiefer liegt als die Kontaktfläche der Elektrode. In anderen Worten ausgedrückt bildet eine Kontaktfläche der Elektroden und/oder der Instrumentenbranchen, die mit Gewebe in Kontakt kommt, eine Ebene aus. Diese Ebene liegt in Kontaktrichtung höher (näher am Gewebe) als die Ebene, auf der die wenigstens eine Beleuchtung und oder der wenigstens eine Detektor angeordnet ist.

Vorzugsweise ermöglicht die (optische) Temperaturmessvorrichtung eine Echtzeit/ online-Bestimmung der Temperatur während eines Sealingvorgangs/ Sealings. Die Online-Bestimmung ist von besonderer Bedeutung für die Güte des Sealings. Die Messung repräsentiert dabei die Temperatur im Gewebe/ die Gewebetemperatur und weist keine Latenzzeit auf oder eine Verfälschung der gemessenen Temperatur durch die Wärmekapazität der Messvorrichtung, beispielsweise durch die Wärmekapazität von Elektroden aus Metall. Der Vorteil einer optischen Temperaturmessung, die sensitiv auf das Wasser im gefassten/ im in Kontakt kommenden Gewebe ist, liegt darin, dass diese Temperaturmessvorrichtung keine nennenswerte Wärmekapazität hat.

Vorzugsweise kann die Remissionsmessung in der Instrumentenbranche bzw. in dem Maulteil eines Seal&Cut Instruments unabhängig von der Position, an der das Gewebe mit der Instrumentenbranche in Kontakt kommt, durchgeführt werden. In anderen Worten ausgedrückt ist die Temperaturmessvorrichtung auf der Oberfläche der Instrumentenbranche in dem Bereich, der dazu vorgesehen und angepasst ist mit dem Gewebe in Kontakt zu kommen, verteilt angeordnet, vorzugsweise gleichmäßig verteilt. Wie vorstehend dargelegt kann die wenigstens eine Instrumentenbranche eine Vielzahl von Anrege- und Detektionspfaden/ Beleuchtungs- oder Detektionspfaden aufweisen, vorzugsweise entlang und/oder in einer Elektrode.

Wie vorstehend ausgeführt wurde soll zusätzlich oder alternativ zu der Messung der Impedanz, eine Messung der Temperatur erfolgen. Die Temperatur wird direkt im zu fusionierenden Gewebe vorzugsweise zwischen zwei sich gegenüberliegenden Instrumentenbranchen gemessen und zwar vorzugsweise im (zeitlichen) Verlauf der Bestromung/Erwärmung des Gewebes. Hierdurch kann die Veränderung des Gewebezustands direkt/online detektiert und somit auch darauf reagiert werden. Durch die Erweiterung des Algorithmus durch einen weiteren Steuer-/ Regelparameter ist es möglich den Energieeintrag in das Gewebe besser zu bewerten und somit die Fusion des Gewebes besser zu steuern/regeln. Zudem können mit der erfindungsgemäßen Temperaturmessvorrichtung auch andere Eigenschaften des Gewebes gemessen werden, zum Beispiel der Wasseranteil/ der Wassergehalt im Gewebe.

Vorzugsweise weist die Elektrode auf der Fläche, die vorgesehen und angepasst ist, mit dem Gewebe in Kontakt zu kommen, wenigstens eine erste Elektrodenoberfläche auf. Die Elektrode befindet sich vorzugsweise auf einem Instrumentenbranchenkörper (im Maulteil) einer Instrumentenbranche oder wird durch die Instrumentenbranche gebildet. In der Elektrode und/oder der Instrumentenbranche sind vorzugsweise wenigstens eine Lichtquelle/ wenigstens ein Lichtleiter/ wenigstens ein optisches Bauteil (dichroitischer Spiegel/ Strahlungsteiler/ Spiegel) und/oder wenigstens ein Lichtdetektor (oder ein Teil davon) mit wenigstens einem Sensor und ggf. einem Lichtleiter eingebracht. Als Sensor kann auch eine Photodiode oder ein Photomultiplier verstanden werden. Die Elektrode weist vorzugsweise wenigstens eine Lichtaustrittsöffnung auf, aus/durch die das Licht der Lichtquelle aus der Elektrodenoberfläche und/oder in das Gewebe strahlt. Die Elektrode weist vorzugsweise wenigstens eine Lichteintrittsöffnung auf, durch die das Licht (ausschließlich) aus dem Gewebe (Remission) in/- durch die Elektrodenoberfläche in den Sensor strahlt/ remittiert/ reflektiert wird. Die Elektrode weist vorzugsweise wenigstens einen Kanal auf, der vorgesehen und angepasst ist, Daten mittels wenigstens eines Kabels/elektrischen Leitung an wenigstens eine Recheneinheit zu leiten, oder Licht, mittels wenigstens eines streuenden Mediums/ wenigstens eines Lichtwellenleiter/ wenigstens einer spiegelnden Oberfläche an einen entfernt gelegenen Sensor zu leiten, der wiederum Daten, mittels wenigstens eines Kabels/elektrischen Leitung an wenigstens eine Recheneinheit leitet. Wenn die Erfindung mehr als eine Elektrodenoberfläche bzw. mehr als eine Instrumentenbranche aufweist, sind die Elektrodenoberflächen/Instrumentenbranchen voneinander beabstandet, vorzugsweise parallel. Der Raum zwischen den Elektrodenoberflächen/Instrumentenbranchen ist vorzugsweise vorgesehen und angepasst, eine Schneidvorrichtung, wie ein Messer, Skalpell, HF-Skalpell oder dergleichen, einschiebbar aufzunehmen, die vorgesehen und angepasst ist, Gewebe zu trennen/schneiden. Auf den wenigstens zwei Seiten des Schnitts des Gewebes sind somit die Elektroden-/Branchenoberflächen ausgeformt, um das Gewebe mittels HF-Technik zu Koagulieren.

Vor dem Sensor ist vorzugsweise ein schmalbandiger Filter angeordnet. Ein Lichttunnel kann in der Elektrode und/oder der Instrumentenbranche gebildet werden. In anderen Worten ausgedrückt kann der Lichttunnel Licht durch die Instrumentenbranche und/oder die wenigstens eine Elektrode führen. Alle Ausführungsformen können untereinander kombinierbar sein.

Die Aufgabe der Erfindung wird hinsichtlich eines medizinischen Instruments erfindungsgemäß dadurch gelöst, dass das medizinische Instrument dafür vorgesehen und angepasst, das erfindungsgemäße Verfahren auszuführen. Insbesondere kann das medizinische Instrument eine Abschaltvorrichtung / eine Vorrichtung zur Bestimmung eines Abschaltzeitpunktes aufweisen, welche dafür vorgesehen und angepasst ist, das erfindungsgemäße Verfahren auszuführen.

**Kurzbeschreibung der Figuren**

Die Erfindung wird nachfolgend anhand bevorzugter Ausführungsformen mit Hilfe von Figuren näher erläutert. Es zeigen:

Fig.1 einen Bereich einer Instrumentenbranche gemäß einer ersten Ausführungsform,

Fig.2 eine erste Beleuchtungs- und Detektionsanordnung einer Instrumentenbranche,

Fig.3 eine zweite Beleuchtungs- und Detektionsanordnung einer Instrumentenbranche,

Fig.4 eine dritte Beleuchtungs- und Detektionsanordnung einer Instrumentenbranche,

Fig.5 einen Bereich einer Instrumentenbranche gemäß einer zweiten Ausführungsform,

Fig.6 die Lichtführung in dem Bereich der Instrumentenbranche gemäß der zweiten Ausführungsform,

Fig.7 einen Bereich einer Instrumentenbranche gemäß einer dritten Ausführungsform,

Fig.8 die Lichtführung in dem Bereich der Instrumentenbranche gemäß der dritten Ausführungsform,

Fig.9 einen Bereich einer Instrumentenbranche gemäß einer vierten Ausführungsform,

Fig.10 die Lichtführung in dem Bereich der Instrumentenbranche gemäß der vierten Ausführungsform,

Fig. 11 einen Bereich einer Instrumentenbranche gemäß einer fünften Ausführungsform,

Fig.12 die Lichtführung in dem Bereich der Instrumentenbranche gemäß der fünften Ausführungsform,

Fig.13 einen Bereich einer Instrumentenbranche gemäß einer sechsten Ausführungsform,

Fig.14 die Lichtführung in dem Bereich der Instrumentenbranche gemäß der sechsten Ausführungsform,

Fig.15 einen Bereich einer Instrumentenbranche gemäß einer siebten Ausführungsform,

Fig. 16 die Lichtführung in dem Bereich der Instrumentenbranche gemäß der sechsten Ausführungsform,

Fig. 17 einen Bereich einer Instrumentenbranche gemäß einer achten Ausführungsform,

Fig. 18 die Lichtführung in dem Bereich der Instrumentenbranche gemäß der achten Ausführungsform,

Fig. 19 einen Bereich einer Instrumentenbranche gemäß einer neunten Ausführungsform,

Fig. 20 die Lichtführung in dem Bereich der Instrumentenbranche gemäß der neunten Ausführungsform,

Fig. 21 einen Bereich einer Instrumentenbranche gemäß einer zehnten Ausführungsform,

Fig. 22 die Lichtführung in dem Bereich der Instrumentenbranche gemäß der zehnten Ausführungsform,

Fig. 23 eine bipolare Instrumentenbranche gemäß vorstehenden Ausführungsformen,

Fig. 24 gegenüberliegende Detektoren und Beleuchtungen auf einem bipolaren HF-Instrument,

Fig. 25 eine schematische Darstellung einer medizinischen Vorrichtung gemäß der Erfindung, und

Fig. 26 eine schematische Darstellung des Temperaturverlaufs während des Verfahrens.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

**Detaillierte Beschreibung bevorzugter Ausführungsformen**

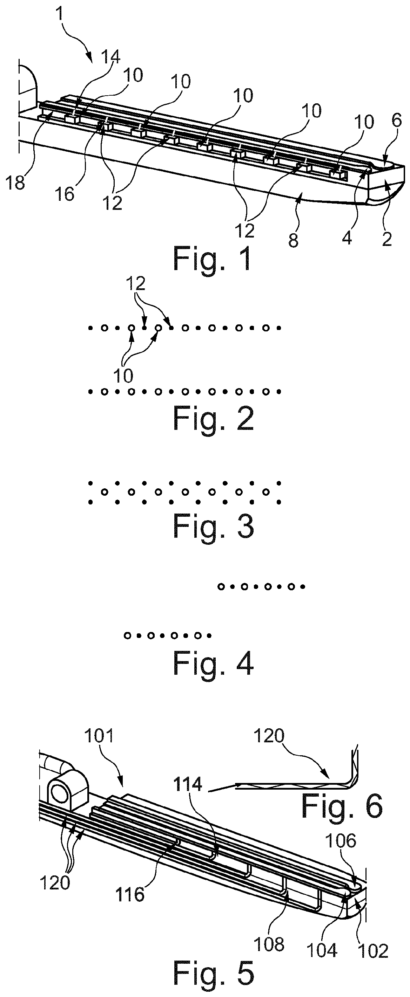

Fig.1 zeigt einen Bereich einer Instrumentenbranche 1 gemäß einer ersten Ausführungsform. Die Instrumentenbranche 1 weist mindestens eine Elektrode 2 auf, welche in die Instrumentenbranche 1 in isolierter Weise eingebettet ist. Die Elektrode 2 weist auf der Branchenseite, die vorgesehen und angepasst ist, mit einem Körpergewebe in Kontakt zu kommen, eine erste Elektrodenoberfläche 4 und eine zweite Elektrodenoberfläche 6 auf. Die Elektrode(n) 2 befindet sich insbesondere in/auf einem Instrumentenbranchenkörper 8 der Instrumentenbranche 1, welcher eine Hälfte eines betätigbaren Instrumentenmaulteils darstellt. In die Elektrode 2 bzw. in die Instrumentenbranche 1/den Instrumentenbranchenkörper 8 sind abwechselnd Lichtquellen (LED's) 10 und Lichtdetektoren bzw. Sensoren 12 eingebracht. Die Elektrode 2 bzw. die Instrumentenbranche 1/der Instrumentenbranchenkörper 8 weist Lichtaustrittsöffnungen 14 auf, durch die das Licht der Lichtquelle 10 aus der Elektrodenoberfläche 4 und/oder 6 bzw. der Branchenkontaktfläche in das Gewebe strahlt. Die Elektrode 2 bzw. die Instrumentenbranche 1/der Instrumentenbranchenkörper 8 weist ferner Lichteintrittsöffnungen 16 auf, durch die das Licht aus dem Gewebe in/ durch die Elektrodenoberfläche 4 und/oder 6 bzw. durch die Branchenkontaktfläche in den Sensor 12 remittiert wird. Die Elektrode 2 bzw. die Instrumentenbranche 1/der Instrumentenbranchenkörper 8 weist wenigstens einen (Längs-)Kanal 18 auf, der vorgesehen und angepasst, ist Daten/Signale von den Sensoren 12 mittels eines Kabels (nicht näher dargestellt) an eine Recheneinheit (nicht näher dargestellt) zu leiten.

Fig.2 zeigt eine erste Variante einer Beleuchtungs- und Detektionsanordnung der Instrumentenbranche 1. Jede der Ausführungsformen dieser Anmeldung kann die erste Beleuchtungs- und Detektionsanordnung aufweisen. Die obere Reihe der Beleuchtungs- und Detektionsanordnung der Figur 2 ist an/in der zweiten Elektroden-/Branchenoberfläche 6 der Fig. 1 angeordnet/eingebettet. Die untere Reihe der Beleuchtungs- und Detektionsanordnung der Figur 2 ist an/in der ersten Elektroden-/Branchenoberfläche 4 der Fig. 1 angeordnet/eingebettet. In den Reihen ist jeweils abwechselnd ein Detektor/ Sensor 12 und eine Beleuchtung/ Lichtquelle 10 angeordnet. Die dunklen Punkte stellen dabei einen Detektor/Sensor 12 und die hellen Punkte eine Beleuchtung/Lichtquelle 10 das. Vor dem Detektor/Sensor 12 ist vorzugsweise ein schmalbandiger (Licht-)Filter (nicht dargestellt) angeordnet. Weiter vorzugsweise sind die optoelektronischen Bauteile (Sensor 12 und Beleuchtung 10) auf einer Platine unterhalb der Elektrode/unterhalb der Gewebekontaktfläche der Branche angebracht.

Fig.3 zeigt eine zweite Variante einer Beleuchtungs- und Detektionsanordnung einer Instrumentenbranche. Jede der Ausführungsformen dieser Anmeldung kann die zweite Variante einer Beleuchtungs- und Detektionsanordnung aufweisen. Die dunklen Punkte stellen dabei einen Sensor 12 und die hellen Punkte eine Lichtquelle 10 dar. Die zweite Variante einer Beleuchtungs- und Detektionsanordnung ist so ausgestaltet, dass um eine Lichtquelle 10 jeweils vier Sensoren 12 in einem gleichen Abstand zur Lichtquelle 10 angeordnet sind, wobei sich die eine Lichtquelle10 jeweils zwei Sensoren 12 mit einer anderen, unmittelbar benachbarten Lichtquelle teilt. Die/jede Lichtquelle 10 befindet sich in anderen Worten im Mittelpunkt eines imaginären Rechtecks, an dessen Eckpunkten die Sensoren 12 positioniert sind.

Fig.4 zeigt eine dritte Variante einer Beleuchtungs- und Detektionsanordnung einer Instrumentenbranche. Jede der Ausführungsformen dieser Anmeldung kann die dritte Variante einer Beleuchtungs- und Detektionsanordnung aufweisen. Die dunklen Punkte stellen dabei einen Sensor 12 und die hellen Punkte eine Lichtquelle 10 dar. Die dritte Variante einer Beleuchtungs- und Detektionsanordnung ist gleich der ersten Variante einer Beleuchtungs- und Detektionsanordnung mit dem Unterschied, dass die Reihe der Beleuchtungs- und Detektionsanordnung der zweiten Elektroden-/Branchenoberfläche dort anfängt, wo die die Reihe der Beleuchtungs- und Detektionsanordnung der ersten Elektroden-/Branchenoberfläche endet.

Fig.5 zeigt einen Bereich einer Instrumentenbranche 101 gemäß einer zweiten Ausführungsform. Die Instrumentenbranche 101 weist eine Elektrode 102 auf. Die Elektrode 102 weist auf der (Branchen-)Fläche, die vorgesehen und angepasst ist, mit dem Gewebe in Kontakt zu kommen, eine erste Elektrodenoberfläche 104 und eine zweite Elektrodenoberfläche 106 auf. Insofern entspricht die Branche der zweiten Ausführungsform der Branche der ersten Ausführungsform. Die Elektrode 102 befindet sich insbesondere auf einem distalen Instrumentenbranchenkörper 108 der Instrumentenbranche 101, der einen Teil eines Instrumenten-Maulteils darstellt. In die Instrumentenbranche 101 sind Lichtquellen und Sensoren (nicht näher dargestellt) entfernt zur Gewebekontaktfläche des Instrumentenbranchenkörpers 108 eingebracht. Die Elektrode 102/Instrumentenbranchenkörper 108 weist Lichtaustrittsöffnungen 114 auf, durch die das Licht der nicht dargestellten Lichtquelle geleitet wird und aus denen Licht aus der Elektrodenoberfläche 104 und/oder 106 bzw. Gewebekontaktfläche des Instrumentenbranchenkörpers 108 in das Gewebe strahlt/ eintritt. Die Elektrode 102/Instrumentenbranchenkörper 108 weist Lichteintrittsöffnungen 116 auf, durch die das Licht aus dem Gewebe in/ durch die Elektrodenoberfläche 104 und/oder 106 bzw. Gewebekontaktfläche des Instrumentenbranchenkörpers 108 in einen Lichttunnel 120 strahlt/ eintritt, der in dem Sensor endet. Auch das Licht von der Lichtquelle zu der Lichtaustrittsöffnung 114 wird durch einen, vorzugsweise anderen, Lichttunnel 120 geleitet. Die Lichttunnel 120 sind mit Luft oder einem anderen Gas gefüllt oder weisen ein Vakuum auf. Die Lichttunnel 120 führen durch den Instrumentenbranchenkörper 108 und/oder durch die Elektrode 102. Die, vorzugsweise zylindrischen Lichttunnel 120 weisen eine innere Tunneloberfläche (in der hohlzylindrischen Form) auf, die wiederum reflektierende Eigenschaften für elektromagnetische Wellen (Lichtwellen) aufweist. Die Tunneloberfläche an der Tunnel-Innenseite ist somit vorgesehen und angepasst, eine Totalreflektion zu ermöglichen.

Fig.6 zeigt die Lichtführung in dem Bereich der Instrumentenbranche/des Instrumentenbranchenkörpers gemäß der zweiten Ausführungsform in dem Lichttunnel 120. Das eintretende Licht von der Lichtquelle kommend wird an der Innenoberfläche des Lichttunnels 120 totalreflektiert und kann somit durch den Lichttunnel 120 geleitet werden. Durch die Totalreflektion an der Innenseite des Lichttunnels 1202 kann das Licht auch durch abgebogene Bereiche/ wenigstens einen Bogen oder dergleichen geführt werden. In diesem Fall ist der Lichttunnel 120 längs des Branchenkörpers 108 geführt um dann in einem im Wesentlichen 90°-Bogen zur Gewebekontaktfläche des Branchenkörpers 108 (oder einem anderen Winkel bezüglich der Gewebekontaktfläche) zu gelangen, wo sich der Lichttunnel 120 öffnet.

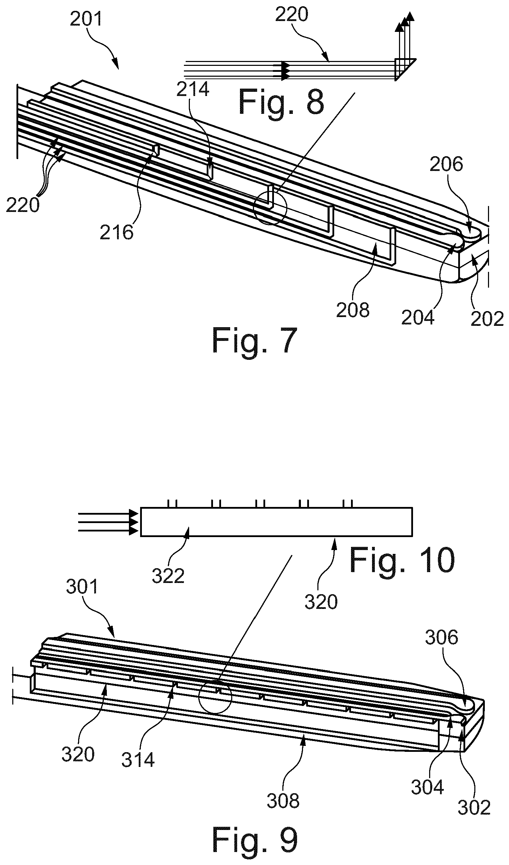

Fig.7 zeigt einen Bereich einer Instrumentenbranche 201 gemäß einer dritten Ausführungsform. Die Instrumentenbranche 201 weist einen Instrumentenbranchenkörper 208 auf, der ein Teil eines Instrumentenmaulteils bildet, der eine Elektrode ist oder in welchem, wie in der Fig. 7 gezeigt, eine Elektrode 202 isolierend eingebettet ist. Die Elektrode 202 weist auf der Branchenfläche, die vorgesehen und angepasst ist, mit dem Gewebe in Kontakt zu kommen, eine erste Elektrodenoberfläche 204 und eine zweite Elektrodenoberfläche 206 auf. Die Elektrode 202 befindet sich demzufolge auf/in dem Instrumentenbranchenkörper 208 der Instrumentenbranche 201. In die Instrumentenbranche 201 sind Lichtquellen und Sensoren entfernt zur Gewebekontaktfläche eingebracht (nicht dargestellt). Die Elektrode 202 bzw. der Instrumentenbranchenkörper 208 weist Lichtaustrittsöffnungen 214 auf, durch die das Licht der nicht dargestellten Lichtquelle geleitet wird und aus denen Licht aus der Elektrodenoberfläche 204 und/oder 206 bzw. der Gewebekontaktfläche in das Gewebe strahlt/ eintritt. Die Elektrode 202 bzw. der Instrumentenbranchenkörper 208 weist Lichteintrittsöffnungen 216 auf, durch die das Licht aus dem Gewebe in/ durch die Elektrodenoberfläche 204 und/oder 206 bzw. die Gewebekontaktfläche des Instrumentenbranchenkörpers 208 in einen Lichttunnel 220 strahlt/ eintritt, der in einem Sensor endet. Auch das Licht von der Lichtquelle zu der Lichteintrittsöffnung 214 wird durch einen, vorzugsweise anderen, Lichttunnel 220 geleitet. Die Lichttunnel 220 sind mit Luft oder einem anderen Gas gefüllt oder weißen ein Vakuum auf. Die Lichttunnel 220 führen durch den Instrumentenbranchenkörper 208 und/oder durch die Elektrode 202. Das Licht der Lichtquelle wird senkrecht zur Öffnung des, vorzugsweise zylindrischen, Lichttunnels 220/ zur Längsrichtung des zylindrischen Lichttunnels 220 eingeleitet/ eingestrahlt. Das Licht wird somit gerade/ geradlinig in dem Lichttunnel 220 geführt. Zur Lenkung des Lichts wird wenigstens ein Spiegel und/oder ein Prisma im Lichttunnel 220 verwendet, um das Licht in einem gewünschten Winkel abzulenken/ zu führen. Der Tunnel 220 kann jedwede geometrische Form annehmen, z.B. zylindrisch, quaderförmig, usw..

Fig.8 zeigt die Lichtführung in dem Bereich der Instrumentenbranche gemäß der dritten Ausführungsform in dem Lichttunnel 220. Das eintretende Licht von der Lichtquelle kommend wird geradlinig/ gerichtet/ parallel gerichtet in den Lichttunnel 220 eingespeist. Durch die Führung mittels wenigstens einem Spiegel in dem Lichttunnel 220 kann das Licht auch über gewinkelte Beiche/ Winkel oder dergleichen geführt werden.

Fig.9 zeigt einen Bereich einer Instrumentenbranche 301 gemäß einer vierten Ausführungsform. Die Instrumentenbranche 301 weist eine Elektrode 302 auf, die in einem Instrumentenbranchenkörper 308 aufgenommen ist, der eine Gewebekontaktfläche ausbildet. Die Elektrode 302 weist auf der Fläche, die vorgesehen und angepasst ist, mit dem Gewebe in Kontakt zu kommen, eine erste Elektrodenoberfläche 304 und eine zweite Elektrodenoberfläche 306 auf. Die Elektrode 302 befindet sich somit in/auf dem Instrumentenbranchenkörper 308 der Instrumentenbranche 301. In der Instrumentenbranche 301 sind entfernt zur Gewebekontaktfläche des Instrumentenbranchenkörpers 308 Lichtquellen und Sensoren eingebracht (nicht dargestellt). Die Elektrode 302 bzw. der Instrumentenbranchenkörper 308 weist Lichtaustrittsöffnungen 314 auf, durch die das Licht der nicht dargestellten Lichtquelle geleitet wird und aus denen Licht aus der Elektrodenoberfläche 304 und/oder 306 bzw. bzw. dem Instrumentenbranchenkörper 308 in das Gewebe strahlt/ eintritt. Die Elektrode 302 bzw. der Instrumentenbranchenkörper 308 weist Lichteintrittsöffnungen (nicht gezeigt) auf, durch die das Licht aus dem Gewebe in/ durch die Elektrodenoberfläche 304 und/oder 306 bzw. durch die Kontaktfläche des Instrumentenbranchenkörpers 308 in einen Lichttunnel 320 strahlt/ eintritt, der in einem Sensor endet. Auch das Licht von der Lichtquelle zu der Lichtaustrittsöffnung 314 wird durch einen, vorzugsweise anderen, Lichttunnel (nicht dargestellt) geleitet. Die Lichttunnel 320 sind mit einem streuenden Bulkmaterial 322 gefüllt. Die Lichttunnel 320 führen durch den Instrumentenbranchenkörper 308 und/oder durch die Elektrode 302. Bei dieser Ausführungsform sind wenigstens zwei Lichttunnel 320 parallel in der Elektrode 302 und/ oder dem Instrumentenbranchenkörper 308 in einer Reihe/ Linie angeordnet, so dass jeweils eine Reihe mit Lichteintrittsöffnungen 314 und Lichtaustrittsöffnungen (nicht gezeigt) in jeweils eine Elektrodenoberfläche 304 und 306 eingebracht ist. In einer nicht gezeigten Ausführungsform kann das Bulk-Material der vierten Ausführungsform selbst eine Lichtquelle darstellen, d.h. das Bulkmaterial kann leuchten.

Fig.10 zeigt die Lichtführung in dem Bereich der Instrumentenbranche gemäß der vierten Ausführungsform in einem Lichttunnel 320. Das eintretende Licht von der Lichtquelle kommend wird in den Lichttunnel 320 eingespeist, genauer gesagt in das streuende und/oder leuchtende Bulkmaterial 322 in dem Lichttunnel 320. Durch die Streuung des Lichts in dem Bulkmaterial 322 wird das Licht in das Gewebe gestrahlt und das remittierte Licht wird von einem anderen Lichttunnel (nicht gezeigt) mit dem gleichen Aufbau zu dem Sensor geleitet/gestreut.

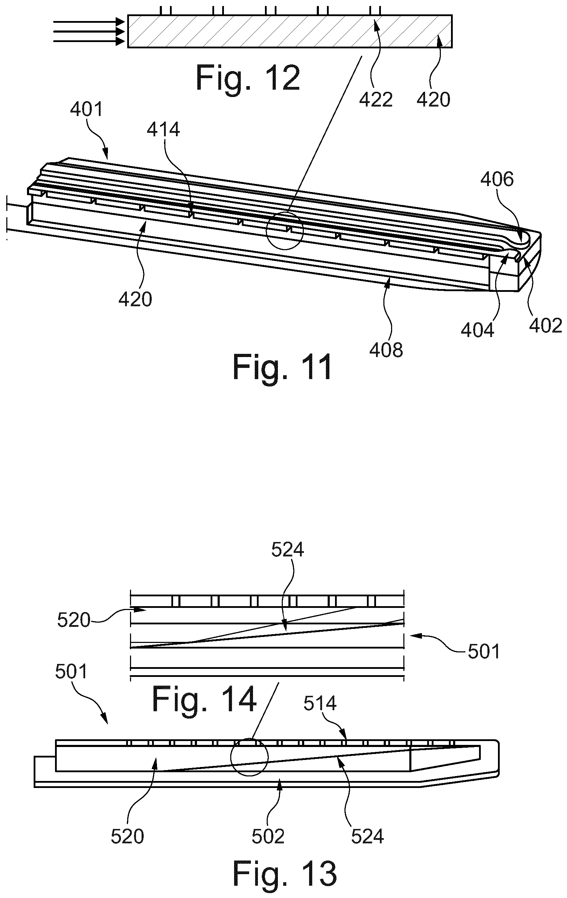

Fig. 11 zeigt einen Bereich einer Instrumentenbranche 401 gemäß einer fünften Ausführungsform. Die Instrumentenbranche 401 weist eine Elektrode 402 auf, die vorliegend in einem Instrumentenbranchenkörper 408 isolierend eingebettet ist. Die Elektrode 402 weist auf der Fläche des Instrumentenbranchenkörpers 408, die vorgesehen und angepasst ist, mit dem Gewebe in Kontakt zu kommen, eine erste Elektrodenoberfläche 404 und eine zweite Elektrodenoberfläche 406 auf. Die Elektrode 402 befindet sich somit in/auf dem Instrumentenbranchenkörper 408 der Instrumentenbranche 401. In die Instrumentenbranche 401 sind Lichtquellen und Sensoren entfernt zur Gewebekontaktfläche des Instrumentenbranchenkörpers 408 eingebracht (nicht dargestellt). Die Elektrode 402 bzw. der Instrumentenbranchenkörper 408 weist Lichtaustrittsöffnungen 414 auf, durch die das Licht einer nicht dargestellten Lichtquelle geleitet wird und aus denen Licht aus der Elektrodenoberfläche 404 und/oder 406 bzw. aus der Gewebekontaktfläche in das Gewebe strahlt/ eintritt. Die Elektrode 402 bzw. der Instrumentenbranchenkörper 408 weist Lichteintrittsöffnungen (nicht gezeigt) auf, durch die das Licht aus dem Gewebe in/ durch die Elektrodenoberfläche 404 und/oder 406 bzw. durch die Gewebekontaktfläche in einen Lichttunnel 420 strahlt/ austritt, der in einem Sensor endet. Auch das Licht von der Lichtquelle zu der Lichtaustrittsöffnung 414 wird durch einen, vorzugsweise anderen, Lichttunnel (nicht dargestellt) geleitet. Die Lichttunnel 420 sind mit einem strukturierten Bulkmaterial 422 gefüllt. Die Lichttunnel 420 führen durch den Instrumentenbranchenkörper 408 und/oder durch die Elektrode 402. Bei dieser Ausführungsform sind wenigstens zwei Lichttunnel 420 parallel in der Elektrode 402 und/ oder dem Instrumentenbranchenkörper 408 in einer Reihe/ Linie angeordnet, so dass jeweils eine Reihe mit Lichteintrittsöffnungen 414 und Lichtaustrittsöffnungen (nicht gezeigt) in jeweils eine Elektrodenoberfläche 404 und 406 eingebracht ist. In einer nicht gezeigten Ausführungsform kann das Bulk-Material der fünften Ausführungsform selbst eine Lichtquelle darstellen, d.h. das Bulkmaterial kann leuchten.

Fig.12 zeigt die Lichtführung in dem Bereich der Instrumentenbranche gemäß der fünften Ausführungsform in einem Lichttunnel 420. Das eintretende Licht von der Lichtquelle kommend wird in den Lichttunnel 420 eingespeist, genauer gesagt in das strukturierte Bulkmaterial 422 in dem Lichttunnel 420. Durch die Struktur des Einsatzes in dem Bulkmaterial 422 wird das Licht in das Gewebe gestrahlt und das remittierte Licht wird von einem anderen Lichttunnel (nicht gezeigt) mit dem gleichen Aufbau zu einem Sensor geleitet/gestreut.

Fig.13 zeigt einen Bereich einer Instrumentenbranche 501 gemäß einer sechsten Ausführungsform. Die Instrumentenbranche 501 weist eine Elektrode 502 auf, wobei in dieser Ausführungsform der Instrumentenbranchenkörper 501 und die Elektrode 502 hinsichtlich ihres Aufbaus und Anordnung den vorhergehenden Ausführungsbeispielen entsprechen. In dem Instrumentenbranchenkörper sind Lichtquellen und Sensoren eingebracht (nicht dargestellt). Die Elektrode 502/ der Instrumentenbranchenkörper weist Lichtaustrittsöffnungen 514 auf, durch die das Licht einer nicht dargestellten Lichtquelle geleitet wird und aus denen Licht in das Gewebe strahlt/ eintritt. Die Elektrode 502/ der Instrumentenbranchenkörper weist Lichteintrittsöffnungen (nicht gezeigt) auf, durch die das Licht aus dem Gewebe in einen Lichttunnel 520 strahlt/ eintritt, der in einem Sensor endet. Das Licht von der Lichtquelle zu der Lichteintrittsöffnung 514 wird durch wenigstens einen Lichttunnel 520 geleitet. Bei dieser Ausführungsform ist ein einziger Lichttunnel 520 in der Elektrode 502 und somit in dem Instrumentenbranchenkörper 501 ausgebildet. Es ist jeweils eine Reihe mit Lichtaustrittsöffnungen 514 und Lichteintrittsöffnungen (nicht gezeigt) in die Elektrode 502 bzw. in den Instrumentenbranchenkörper eingebracht. **In** dem Lichttunnel 520 ist wenigstens eine verspiegelte/ reflektierende schiefe/ angewinkelte Ebene 524 ausgeformt. Die Ebene 524 kann durch Polieren der Elektrode bzw. des Instrumentenbranchenköpers hergestellt werden oder indem ein Spiegel in den Lichttunnel 520 eingebracht wird. Der Lichttunnel 520 führt durch den Instrumentenbranchenkörper. Es ist wenigstens eine Reihe mit Lichtaustrittsöffnungen 514 und Lichteintrittsöffnungen (nicht gezeigt) in einer Oberfläche der Elektrode 502 / des Instrumentenbranchenkörpers eingebracht. Alternativ oder zusätzlich kann ein einziger Lichttunnel 520 dieser Art sowohl für die Anregung als auch für die Aufnahme von reflektiertem Licht dienen - mit den entsprechenden Filtern. Das bedeutet, dass nach der Lichtquelle ein Filter angebracht ist, der dem Remissionwellenlängenbereich entspricht, das restliche Licht wird aber in das Gewebe geleitet und von derselben und/oder einer benachbarten Öffnung aufgenommen und über dieselbe reflektierende Ebene 524 zurück an den Sensor geführt.

Fig.14 zeigt die Lichtführung in dem Bereich der Instrumentenbranche 501 gemäß der sechsten Ausführungsform in dem Lichttunnel 520. Das eintretende Licht von der Lichtquelle kommend wird in den Lichttunnel 520 eingespeist und an der angewinkelten, spiegelnden Ebene 524 in einem vorbestimmten Winkel (vorzugsweise mit einem Winkel zwischen 0° und 90°) abgelenkt. Durch den/ die Spiegel/ spiegelnde Oberfläche/ spiegelnde Ebene 524 wird das Licht in das Gewebe gestrahlt und das remittierte Licht wird von einem anderen Lichttunnel (nicht gezeigt) mit dem gleichen Aufbau zu einem Sensor geleitet/ geführt.

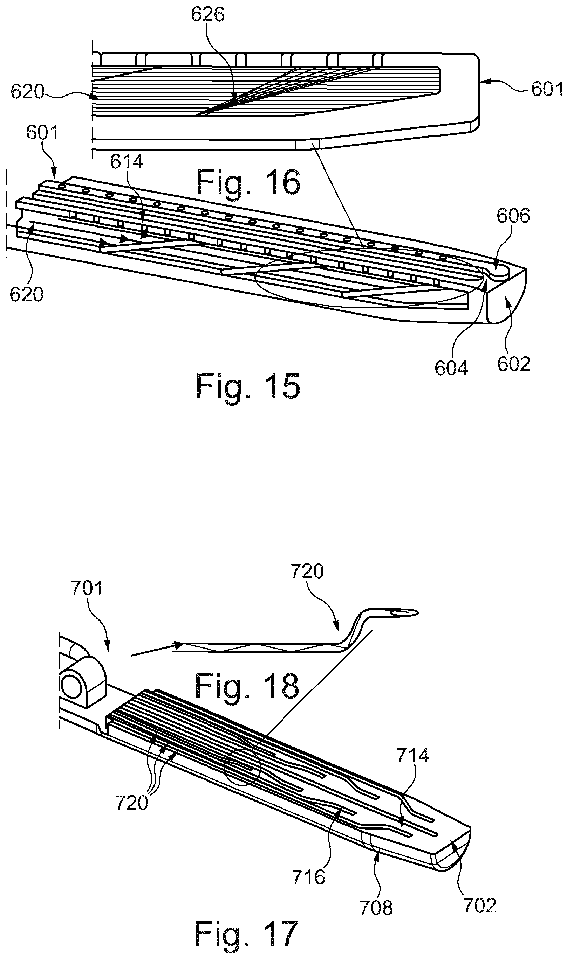

Fig. 15 zeigt einen Bereich einer Instrumentenbranche 601 gemäß einer siebten Ausführungsform. Die Instrumentenbranche 601 weist eine von einem Instrumentenbranchenkörper 608 aufgenommene Elektrode 602 auf. Die Elektrode 602 weist auf der Fläche des Instrumentenbranchenkörpers 608, die vorgesehen und angepasst ist, mit dem Gewebe in Kontakt zu kommen, eine erste Elektrodenoberfläche 604 und eine zweite Elektrodenoberfläche 606 auf. In dem Instrumentenbranchenkörper 608 sind Lichtquellen und Sensoren eingebracht (nicht dargestellt). Der Instrumentenbranchenkörper 608 weist Lichtaustrittsöffnungen 614 auf, durch die das Licht einer nicht dargestellten Lichtquelle geleitet wird und aus der Gewebekontaktfläche in das Gewebe strahlt/ eintritt. Der Instrumentenbranchenkörper 608 weist ferner Lichteintrittsöffnungen (nicht gezeigt) auf, durch die das Licht aus dem Gewebe in/ durch die Gewebekontaktfläche des Instrumentenbranchenkörpers 608 in einen Lichttunnel 620 strahlt/ eintritt, der in einem Sensor endet. Auch das Licht von der Lichtquelle zu der Lichtaustrittsöffnung 614 wird durch einen zweiten Lichttunnel (nicht dargestellt) geleitet. In den Lichttunnel 620 ist wenigstens eine teil-lichtdurchlässige Ebene 626 eingebracht, die einen Teil einer elektromagnetischen Strahlung transmittiert, also für einen Teil des Lichts durchlässig ist und ein Teil des Lichts reflektieren.

Vorzugsweise ist die teil-lichtdurchlässige Ebene ein teil-lichtdurchlässiger Spiegel und weiter bevorzugt sind in dem Lichttunnel mehrere teil-lichtdurchlässige Ebenen 626 hintereinander angeordnet.

Fig.16 zeigt die Lichtführung in dem Bereich der Instrumentenbranche 601 gemäß der siebten Ausführungsform in einem Lichttunnel 620. Das eintretende Licht von der Lichtquelle kommend wird in den Lichttunnel 620 eingespeist. Das eintretende Licht von der Lichtquelle kommend wird geradlinig/ gerichtet/ parallel gerichtet in den Lichttunnel 620 eingespeist. Durch die Führung mittels wenigstens eines teil-lichtdurchlässigen Spiegels 626 in dem Lichttunnel 620 wird das Licht über gewinkelte Bereiche/ Winkel oder dergleichen geführt/ reflektiert/ gespiegelt. Das Licht, das einen teil-lichtdurchlässigen Spiegel 626 durchdringt, trifft auf einen weiteren teil-lichtdurchlässigen Spiegel 626, der in dem gleichen Winkel wie der der vorherige Spiegel angeordnet ist und so weiter. Durch den teil-lichtdurchlässigen Spiegel/ spiegelnde Oberfläche/ spiegelnde Ebene 626 wird das Licht in das Gewebe gestrahlt und das remittierte Licht wird von einem anderen Lichttunnel (nicht gezeigt) mit dem gleichen Aufbau zu einem Sensor geleitet/ geführt.

Fig.17 zeigt einen Bereich einer Instrumentenbranche 701 gemäß einer achten Ausführungsform. Die Instrumentenbranche 701 weist eine Elektrode 702 auf. Die Elektrode 702 befindet sich auf einem Instrumentenbranchenkörper 708 der Instrumentenbranche 701. In die Instrumentenbranche 701 sind Lichtquellen und Sensoren entfernt zum Instrumentenbranchenkörper 708 eingebracht, vorzugsweise extern (nicht dargestellt). Der Instrumentenbranchenkörper 708 weist wenigstens einen Lichttunnel 720 auf, durch den das Licht der nicht dargestellten Lichtquelle geleitet wird und aus dem Licht in das Gewebe strahlt/ eintritt. Die Instrumentenbranchenkörper 708 weist wenigstens einen weiteren Lichttunnel 720 auf, durch den das Licht aus dem Gewebe zu einem Sensor geführt wird. Die Lichttunnel 720 werden in dieser Ausführungsform von Lichtwellenleitern wie zum Beispiel Glasfasern gebildet.

Fig.18 zeigt die Lichtführung in dem Bereich der Instrumentenbranche gemäß der achten Ausführungsform in einem Lichttunnel 720. Das eintretende Licht von der Lichtquelle kommend wird an der Innenoberfläche des Lichttunnels 720 totalreflektiert und kann somit durch den Lichttunnel 720 geleitet werden. Durch die Totalreflektion an der Innenseite des Lichttunnels 720 kann das Licht auch durch abgebogene Bereiche/ wenigstens einen Bogen oder dergleichen geführt werden.

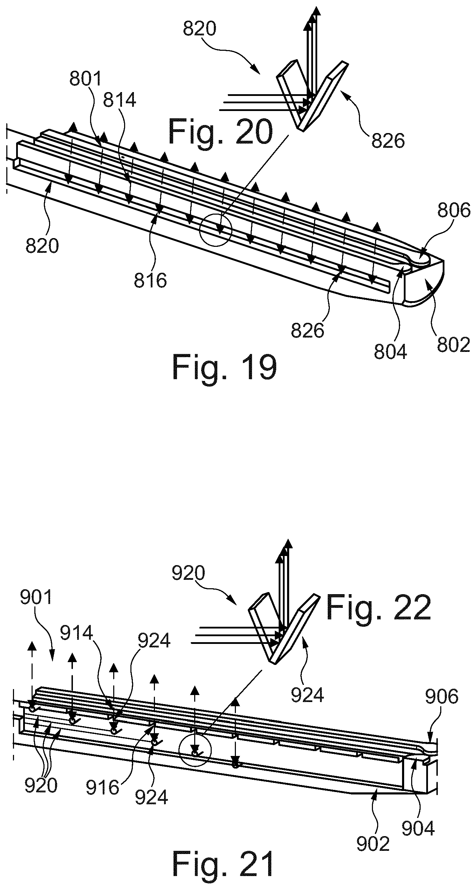

Fig. 19 zeigt einen Bereich einer Instrumentenbranche 801 gemäß einer neunten Ausführungsform. Die Instrumentenbranche 801 weist eine Elektrode 802 auf. Die Elektrode 802 weist auf der Gewebekontaktfläche ihres Instrumentenbranchenkörpers, die vorgesehen und angepasst ist, mit dem Gewebe in Kontakt zu kommen, eine erste Elektrodenoberfläche 804 und eine zweite Elektrodenoberfläche 806 auf. In dem Instrumentenbranchenkörper sind Lichtquellen und Sensoren eingebracht (nicht dargestellt). Der Instrumentenbranchenkörper weist zudem Lichtaustrittsöffnungen 814 auf, durch die das Licht einer nicht dargestellten Lichtquelle geleitet wird und in das Gewebe strahlt/ eintritt. Der Instrumentenbranchenkörper weist auch Lichteintrittsöffnungen 816 auf, durch die das Licht aus dem Gewebe in/ durch den Instrumentenbranchenkörper in einen Lichttunnel 820 strahlt/ eintritt, der in einem Sensor endet. Das Licht von der Lichtquelle zu der Lichtaustrittsöffnung 814 wird durch denselben Lichttunnel geleitet. In anderen Worten ausgedrückt, können Lichtaustrittsöffnungen 814 als Lichteintrittsöffnungen 816 fungieren und vice versa. In den Lichttunnel 820 sind wenigstens zwei teil-lichtdurchlässige Ebenen 626 eingebracht, die einen Teil einer elektromagnetischen Strahlung transmittieren, also für einen Teil des Lichts durchlässig sind, und einen Teil des Lichts reflektieren. Vorzugsweise ist die teildurchlässige Ebene ein teildurchlässiger Spiegel und weiter bevorzugt sind in dem Lichttunnel mehrere teildurchlässige Ebenen 626 hintereinander angeordnet. Durch diese Anordnung in dieser Ausführungsform ist jeweils ein teildurchlässiger Spiegel jeweils einer Lichtaustrittsöffnung 814 oder einer Lichteintrittsöffnung 816 zugeordnet.

Fig.20 zeigt die Lichtführung in dem Bereich der Instrumentenbranche 801 gemäß der neunten Ausführungsform in einem Lichttunnel 820. Das eintretende Licht von der Lichtquelle kommend wird in den Lichttunnel 820 eingespeist. Das eintretende Licht von der Lichtquelle kommend wird geradlinig/ gerichtet/ parallel gerichtet in den Lichttunnel 820 eingespeist. Durch die Führung mittels wenigstens zweier teil-lichtdurchlässigen Spiegel 826 in dem Lichttunnel 820 wird das Licht über gewinkelte Bereiche/ Winkel oder dergleichen geführt/ reflektiert/ gespiegelt. Das Licht, das einen teil-lichtdurchlässigen Spiegel 826 durchdringt, trifft auf einen wenigstens einen weiteren teil-lichtdurchlässigen Spiegel 826, der in dem gleichen Winkel wie der vorherige Spiegel angeordnet ist und so weiter. Durch den teil-lichtdurchlässigen Spiegel/ spiegelnde Oberfläche/ spiegelnde Ebene 826 wird das Licht in das Gewebe gestrahlt und das remittierte Licht wird von demselben Lichttunnel 820 aber durch eine benachbarte Öffnung zu einem Sensor geleitet/ geführt. In wieder anderen Worten ist eine Öffnung zugleich Lichtaustrittsöffnung und Lichteintrittsöffnung für eine benachbarte Öffnung.

Fig.21 zeigt einen Bereich einer Instrumentenbranche 901 gemäß einer zehnten Ausführungsform. Die Instrumentenbranche 901 weist eine Elektrode 902 auf, wobei in dieser Ausführungsform der Instrumentenbranchenkörper sowie die Elektrode hinsichtlich ihres Aufbaus und Anordnung den vorhergehenden Ausführungsbeispielen entsprechen. In der Instrumentenbranche sind demzufolge Lichtquellen und Sensoren entfernt zu der Gewebekontaktfläche des Instrumentenbranchenkörpers eingebracht (nicht dargestellt). Der Instrumentenbranchenkörper weist Lichtaustrittsöffnungen 914 auf, durch die das Licht einer nicht dargestellten Lichtquelle geleitet wird und aus denen Licht in das Gewebe strahlt/ eintritt. Der Instrumentenbranchenkörper weist ferner Lichteintrittsöffnungen 916 auf, durch die das Licht aus dem Gewebe in einen Lichttunnel 920 strahlt/ austritt, der in einem Sensor endet. Das Licht von der Lichtquelle zu der Lichtaustrittsöffnung 914 wird durch wenigstens einen 920 Lichttunnel geleitet. Das Licht von der Lichteintrittsöffnung 91 zu dem Sensor wird durch wenigstens einen weiteren Lichttunnel 920 (der gleichen Bauart) geleitet. Bei dieser Ausführungsform sind somit wenigstens zwei Lichttunnel 920 in dem Instrumentenbranchenkörper ausgebildet. Die Lichtaustrittsöffnung/en 914 und Lichteintrittsöffnung/en 916 sind abwechselnd in den Instrumentenbranchenkörper eingebracht. In dem Lichttunnel 920 ist wenigstens eine verspiegelte/ reflektierende schiefe/ angewinkelte Ebene 924 ausgeformt.

Fig.22 zeigt die Lichtführung in dem Bereich der Instrumentenbranche 901 gemäß der zehnten Ausführungsform in dem Lichttunnel 920. Das eintretende Licht von der Lichtquelle kommend wird in den Lichttunnel 920 eingespeist, und an der angewinkelten spiegelnden Ebene 924 in einem vorbestimmten Winkel (vorzugsweise mit einem Winkel zwischen 0 und 90°) abgelenkt. Durch den/ die Spiegel/ spiegelnde Oberfläche/ spiegelnde Ebene 924 wird das Licht in das Gewebe gestrahlt und das remittierte Licht wird von einem anderen Lichttunnel 924 mit dem gleichen Aufbau zu einem Sensor geleitet/ geführt.

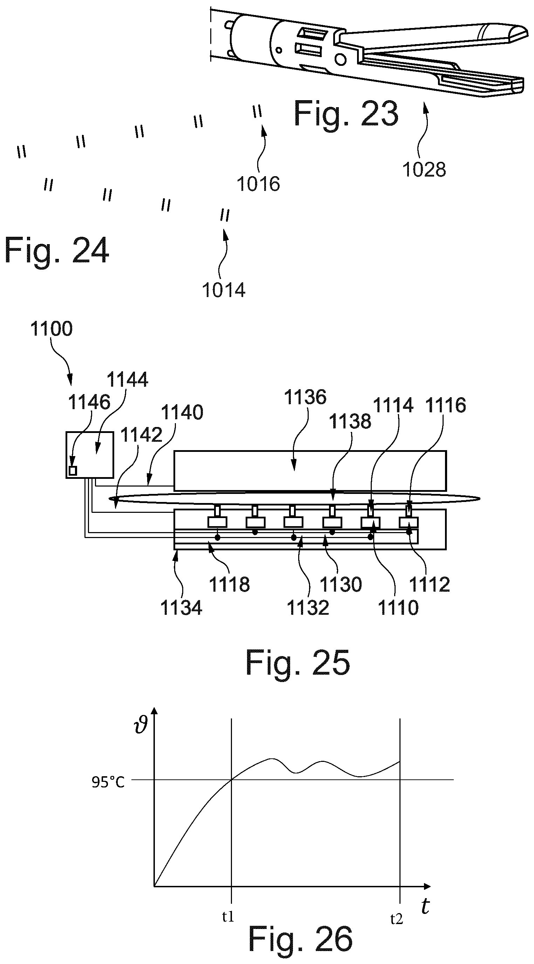

Fig. 23 zeigt eine bipolare Instrumentenbranche gemäß vorstehender Ausführungsformen. Die Ausführungsformen eins bis zehn sind vorgesehen und angepasst, in einem bipolaren medizinischen HF-Instrument verwendet werden, bei welchem zwei Instrumentenbranchenkörper vorzugsweise schwenkbar zueinander gelagert sind und zwischen sich einen Gewebeaufnahmespalt definieren.

Fig. 24 zeigt sich gegenüberliegende Detektoren und Beleuchtungen an einem bipolaren HF-Instrument. Dabei sind der die Lichtaustrittsöffnungen 1014 der Beleuchtungen und die Lichteintrittsöffnungen 1016 der Detektoren jeweils auf gegenüberliegenden Instrumentenbranchen/ Instrumentenbranchenkörpern angeordnet.

Fig. 25 zeigt eine schematische Darstellung einer medizinischen Vorrichtung 1100 gemäß der Erfindung. Eine Lichtquelle 1110 ist dafür vorgesehen und angepasst, Licht auszustrahlen. Ein Sensor 1112 ist dafür vorgesehen und angepasst, Licht zu detektieren. Die Lichtquelle strahlt das Licht durch eine Lichtaustrittsöffnung 1114. Der Sensor 1112 empfängt Licht über eine Lichteintrittsöffnung 1116. Die Lichtquellen 1110 und die Sensoren 1112 sind in Verbindung mit in einem Kanal 1118 befindlichen Datenleitungen 1130 und 1132. Der Kanal 1118 ist in einem Instrumentenbranchenkörper ausgebildet, die auch die Elektroden isolierend aufnimmt. Der einen Instrumentenbranchenkörper, in welcher die Elektrode 1134 aufgenommen ist, klemmt mit einem Instrumentenbranchenkörper, in welchem die gegenüberliegende Elektrode 1136 aufgenommen ist, das Gewebe 1138 ein. Die Elektrode 1134 und die Elektrode 1136 sind mit den Leitungen 1140 und 1142 in Verbindung. Die Datenleitungen 1130 und 1132, sowie die Leitungen 1140 und 1142 sind in Verbindung mit einer Recheneinheit 1144, die ein Speichermedium 1146 aufweist.

Fig. 26 zeigt eine schematische Darstellung eines Temperaturverlaufs während dem Verfahren. Dabei ist die Temperatur v des Gewebes über die Zeit t in einer Graphik aufgetragen. t1 markiert den Zeitpunkt, bei dem das Gewebe die Temperatur von 85°Celsius, vorzugsweise 95° Celsius, erreicht. T2 markiert den Punkt, an dem der Wert SP einen Wert von 2,5 erreicht. Der Wert SP wurde online ab dem Zeitpunkt berechnet, an dem die Temperatur des Gewebes 85°Celsius, vorzugsweise 95°, erreicht hat. Ab diesem Zeitpunkt wurde die die Dauer bei der die Temperatur eines Gewebes über 85°Celsius, vorzugsweise über 95° Celsius, liegt gemessen und online die mittlere Temperatur ab dem erstmaligen Erreichen von 85°Celsius, vorzugsweise 95°Celsius, berechnet (hier dargestellt mit der gestrichelten Linie). Diese beiden Werte werden in Echtzeit multipliziert und durch den Energieeintrag bis zum Erreichen von 85°Celsius, vorzugsweise 95°Celsius dividiert, wodurch der Wert SP berechnet wird, vorzugsweise in Echtzeit.

**Bezugszeichenliste**

1, 101, 201, 301, 401, 501, 601, 701, 801, 901 Instrumentenbranche
2, 102, 202, 302, 402, 502, 602, 702, 802, 902 Elektrode
4, 104, 204, 304, 404, 604, 804, 904 Erste Elektrodenoberfläche
6, 106, 206, 306, 406, 606, 806, 906 Zweite Elektrodenoberfläche
8, 108, 208, 308, 408, 708 Instrumentenbranchenkörper
10, 1110 Lichtquelle
12, 1112 Sensor
14, 114, 214, 314, 414, 514, 614, 714, 814, 914, 1014, 1114 Lichteintrittsöffnung
16, 116, 216, 616, 716, 816, 916, 1016, 1116 Lichtaustrittsöffnung
18, 1118 Kanal
120, 220, 320, 420, 520, 620, 720, 820, 920 Lichttunnel
322, 422 Bulk-Material
524, 924 Verspiegelte schiefe Ebene
626, 826 Teildurchlässige Eben
1028 Bipolare Instrumentenbranche
1130, 1132, 1140, 1142 Leitungen

1134 Erste Elektrode
1136 Zweite Elektrode
1138 Gewebe
1144 Recheneinheit
1146 Speichermedium

**Patentansprüche**

1. Computerlesbares Speichermedium eines medizinischen Instruments mit einer Temperaturmessvorrichtung zur Messung der Temperatur eines Gewebes, vorzugsweise eines medizinischen Instruments gemäß Anspruch 11, auf dem ein Verfahren zur Bestimmung eines Abschaltzeitpunktes des medizinischen Instruments aufweisend die Schritte:

   • Messen einer zeitlichen Dauer, bei der eine Temperatur eines Gewebes über 85°Celsius, vorzugsweise über 95° Celsius, liegt und bevorzugt unter 110° Celsius, vorzugsweise unter 100° Celsius liegt,
   • Berechnen, vorzugsweise online, einer mittleren Temperatur ab dem erstmaligen Erreichen der Temperatur von 85° Celsius, vorzugsweise von 95° Celsius und bevorzugt unter 110° Celsius, vorzugsweise unter 100° Celsius,
   • Messen und/oder Berechnen eines Energieeintrags bis zum Erreichen der Temperatur von 85° Celsius, vorzugsweise bis zum Erreichen der Temperatur von 95° Celsius und bevorzugt unter 110° Celsius, vorzugsweise unter 100° Celsius,
   • Berechnen eines Parameters SP, der die vorstehenden Ergebnisse verknüpft und bei einem vorbestimmten Wert des Parameters SP, vorzugsweise bei einem vorbestimmten Wert SP zwischen 2 und 3, insbesondere bei einem vorbestimmten Wert SP von 2.5, Abschalten des medizinischen Instruments

   gespeichert ist und bei der Ausführung durch einen Computer diesen veranlassen, das Verfahren auszuführen, wobei der Parameter SP das Ergebnis aus der Dauer, bei der die Temperatur des Gewebes über 85° Celsius, vorzugsweise über 95° Celsius und bevorzugt unter 110° Celsius, vorzugsweise unter 100° Celsius, liegt multipliziert mit der mittleren Temperatur ab dem erstmaligen Erreichen der Temperatur von 85° Celsius, vorzugsweise 95° Celsius und bevorzugt unter 110° Celsius, vorzugsweise unter 100° Celsius, dividiert durch den Energieeintrag bis zum Erreichen der Temperatur von 85° Celsius, vorzugsweise 95° Celsius, ist.

2. Speichermedium nach Anspruch 1, **dadurch gekennzeichnet, dass** das Verfahren ferner die Schritte aufweist

• Aussenden von Licht, mit einem Anregungsspektrum, in ein Gewebe mittels wenigstens einer Beleuchtung (10, 1110),
• Empfangen der Remission des Lichtes, mit einem Remissionsspektrum, aus dem Gewebe durch wenigstens einen Detektor (12, 1112),
• Umwandeln des Remissionsspektrums mittels des Detektors (12, 1112) in ein Detektorsignal,
• Senden des Detektorsignals an eine Recheneinheit (1144),
• Berechnen des Remissionsspektrums aus dem Detektorsignal mittels der Recheneinheit (1144),
• Berechnen eines Absorptionsspektrums des Gewebes durch Vergleich des Anregungsspektrums mit dem Remissionsspektrum mittels der Recheneinheit (1144),
• Berechnen wenigstens eines Absorptionsmaximums aus dem Absorptionsspektrum mittels der Recheneinheit (1144), und
• Berechnen einer Temperatur in dem Gewebe durch Vergleich des Absorptionsmaximums mit wenigstens einer Referenz mittels der Recheneinheit (1144)

3. Speichermedium nach einem der vorstehenden Ansprüche, **dadurch gekennzeichnet, dass** das Verfahren ferner den Schritt aufweist

• Speichern wenigstens einer Referenz in Form eines Absorptionsmaximums bei einer bestimmten Temperatur in der Recheneinheit, vorzugsweise einem Speichermedium in der Recheneinheit, vorzugsweise für Wasser und/oder Fett und/oder Kollagen.

4. Speichermedium nach einem der vorstehenden Ansprüche, **dadurch gekennzeichnet, dass** das Verfahren ferner den Schritt aufweist

• Aufbringen der Beleuchtung und des Detektors (12, 1120) auf das Gewebe.

5. Speichermedium nach einem der vorstehenden Ansprüche, **dadurch gekennzeichnet, dass** das Verfahren ferner den Schritt aufweist

• Steuern und/oder Regeln und/oder Abschalten einer Vorrichtung, vorzugsweise eines medizinischen Instruments, mittels der Recheneinheit auf Basis der berechneten Temperatur und/oder Gewebeimpedanz.

6. Speichermedium nach Anspruch 5, **dadurch gekennzeichnet, dass** das Steuern und/oder Regeln und/oder Abschalten bei Erreichen einer vorbestimmten Temperatur, vorzugsweise bei einer Temperatur größer als 85°Celsius, vorzugsweise größer als 95° Celsius, und vorzugsweise kleiner als 100° Celsius, stattfindet.

7. Speichermedium nach Anspruch 5 oder 6, **dadurch gekennzeichnet, dass** das Steuern und/oder Regeln und/oder Abschalten eines medizinischen Instruments online, vorzugsweise in Echtzeit erfolgt.

8. Speichermedium nach einem der vorstehenden Ansprüche, **dadurch gekennzeichnet, dass** das Verfahren zur Temperaturmessung während eines Sealingvorgangs durchgeführt wird.

9. Speichermedium nach einem der Ansprüche 2 bis 8, **dadurch gekennzeichnet, dass** die Detektoren vorgesehen und angepasst sind, Remission, vorzugsweise die Remissionsspektren, im NIR-Bereich von 1000nm bis 1700nm, besonders bevorzugt im Bereich von 1400nm bis 1600nm, zu messen.

10. Speichermedium nach einem der vorstehenden Ansprüche, **dadurch gekennzeichnet, dass** die Beleuchtung und der Detektor beabstandet sind.

11. Medizinisches Instrument (1110) zum Versiegeln und/oder Schneiden von Gewebe,

mit einer Temperaturmessvorrichtung, welche dafür vorgesehen und angepasst ist, eine Temperatur eines Gewebes zu messen, und mit einer Recheneinheit (1144),
wobei das medizinische Instrument (1110), insbesondere die Temperaturmessvorrichtung, dafür vorgesehen und angepasst ist, eine zeitliche Dauer bei der die Temperatur des Gewebes über 85°Celsius, vorzugsweise über 95° Celsius, liegt und bevorzugt unter 110° Celsius, vorzugsweise unter 100° Celsius, liegt, zu messen,
wobei die Recheneinheit (1144) dafür vorgesehen und angepasst ist, vorzugsweise online, eine mittlere Temperatur ab dem erstmaligen Erreichen der Temperatur von 85° Celsius, vorzugsweise ab dem erstmaligen Erreichen der Temperatur von 95° Celsius und bevorzugt unter 110° Celsius, vorzugsweise unter 100° Celsius, zu berechnen,
wobei das medizinische Instrument (1110) ferner dafür angepasst ist, einen Energieeintrag bis zum Erreichen der Temperatur von 85° Celsius, vorzugsweise von 95° Celsius und bevorzugt unter 110° Celsius, vorzugsweise unter 100° Celsius, zu messen und/oder zu berechnen,
wobei die Recheneinheit (1144) dafür vorgesehen und angepasst ist, einen Parameter SP, der die vorstehenden Ergebnisse der zeitlichen Dauer, der mittleren Temperatur und dem Energieeintrag verknüpft, zu berechnen, und wobei das medizinische Instrument (1110), insbesondere die Recheneinheit (1144), dafür angepasst ist, bei und/oder ab einem vorbestimmten Wert SP, vorzugsweise bei einem vorbestimmten Wert SP zwischen 2 und 3, insbesondere bei und/oder ab einem Wert SP von 2.5, das medizinische Instrument (1110) abzuschalten,

wobei der Parameter SP das Ergebnis aus der Dauer, bei der die Temperatur des Gewebes über 85° Celsius, vorzugsweise über 95° Celsius und bevorzugt unter 110° Celsius, vorzugsweise unter 100° Celsius, liegt multipliziert mit der mittleren Temperatur ab dem erstmaligen Erreichen der Temperatur von 85° Celsius, vorzugsweise 95° Celsius und bevorzugt unter 110° Celsius, vorzugsweise unter 100° Celsius, dividiert durch den Energieeintrag bis zum Erreichen der Temperatur von 85° Celsius, vorzugsweise 95° Celsius, ist.

12. Medizinisches Instrument (1110) nach Anspruch 11, **dadurch gekennzeichnet, dass** das medizinische Instrument (1110) wenigstens eine Instrumentenbranche (1, 101, 201, 301, 401, 501, 601, 701, 801, 901) aufweist, die wenigstens eine bestrombare Elektrode (502, 602, 702, 802, 902,1134,1136) zum Versiegeln und/oder Schneiden von Gewebe bildet oder in oder an der wenigstens eine bestrombare Elektrode (2, 102, 202, 302, 402, 702) zum Versiegeln und/oder Schneiden von Gewebe angeordnet ist, wobei die Bestromung der Elektrode (2, 102, 202, 302, 402, 502, 602, 702, 802, 902, 1134,1136) durch die Recheneinheit (1144) steuerbar und/oder regelbar ist.

**Claims**

1. A computer-readable storage medium of a medical instrument with a temperature measurement device for measuring the temperature of a tissue, preferably of a medical instrument according to claim 11, having a method for determination of a switch-off time of a medical instrument stored thereon, comprising the steps of:

   • measuring a duration for which the temperature of a tissue is above 85° Celsius, preferably above 95° Celsius, and preferably below 110° Celsius, preferably below 100° Celsius,
   • calculating, preferably online, a mean temperature as from the first time when the temperature reaches 85° Celsius, preferably 95° Celsius, and preferably below 110° Celsius, preferably below 100° Celsius,
   • measuring and / or calculating an energy input until the temperature of 85° Celsius is reached, preferably until the temperature of 95° Celsius is reached, and preferably below 110° Celsius, preferably below 100° Celsius,
   • calculating a parameter SP, which links the above-mentioned results, and at a predetermined value of the parameter SP, preferably at a predetermined value SP between 2 and 3, in particular at a predetermined value SP of 2.5, switch-off of the medical instrument

and, when executed by a computer, cause the computer to carry out the method, wherein the parameter SP is the result from the duration for which the temperature of the tissue is above 85° Celsius, preferably above 95° Celsius, and preferably below 110° Celsius, preferably below 100° Celsius, multiplied by the mean temperature as from the first time when the temperature of 85° Celsius is reached, preferably 95° Celsius, and preferably below 110° Celsius, preferably below 100° Celsius, divided by the energy input until the temperature of 85° Celsius, preferably 95° Celsius, is reached.

2. The storage medium according to claim 1, **characterized in that** the method moreover comprises the steps of

   • emitting light with an excitation spectrum into a tissue by means of at least one illumination (10, 1110),
   • receiving the remission of light with a remission spectrum from the tissue by at least one detector (12, 1112),
   • converting the remission spectrum by means of the detector (12, 1112) into a detector signal,
   • sending the detector signal to a calculating unit (1144),
   • calculating the remission spectrum from the detector signal by means of the calculating unit (1144),
   • calculating an absorption spectrum of the tissue by comparing the excitation spectrum with the remission spectrum using the calculating unit (1144),
   • calculating at least one absorption maximum from the absorption spectrum by means of the calculating unit (1144), and
   • calculating a temperature in the tissue by comparing the absorption maximum with at least one reference by means of the calculating unit (1144)

3. The storage medium according to one of the preceding claims, **characterized in that** the method moreover comprises the step of

   • storing at least one reference in the form of an absorption maximum at a certain temperature in the calculating unit, preferably a storage medium in the calculating unit, preferably for water and / or fat and / or collagen.

4. The storage medium according to one of the preceding claims, **characterized in that** the method moreover comprises the step of

> • applying the illumination and the detector (12, 1120) to the tissue.

5. The storage medium according to one of the preceding claims, **characterized in that** the method moreover comprises the step of

> • controlling and / or adjusting and / or switching off a device, preferably a medical instrument, by means of the calculating unit based on the calculated temperature and / or tissue impedance.

6. The method according to claim 5, **characterized in that** the controlling and / or adjusting and / or switching off takes place when a predetermined temperature is reached, preferably at a temperature that is greater than 85° Celsius, preferably greater than 95° Celsius, and preferably less than 100° Celsius.

7. The storage medium according to claim 5 or 6, **characterized in that** the controlling and / or adjusting and / or switching off of a medical instrument takes place online, preferably in real time.

8. The storage medium according to one of the preceding claims, **characterized in that** the method for temperature measurement is performed during a sealing process.

9. The storage medium according to any one of claim 2 to 8, **characterized in that** the detectors are provided and adapted to measure remission, preferably the remission spectra, in the NIR range of 1000 nm to 1700 nm, more preferable in the range of 1400 nm to 1600 nm.

10. The storage medium according to one of the preceding claims, **characterized in that** the illumination and the detector are spaced apart.

11. A medical instrument (1110) for sealing and / or cutting of tissue,

> comprising a temperature measurement device which is provided and adapted to measure a temperature of a tissue, and
> comprising a calculating unit (1144),
> wherein the medical instrument (1110), in particular the temperature measurement device is provided and adapted to measure a duration for which the temperature of the tissue is above 85° Celsius, preferably above 95° Celsius, and preferably below 110° Celsius, preferably below 100° Celsius,
wherein the calculating unit (1144) is provided and adapted to calculate, preferably online, a mean temperature as from which the temperature of 85° Celsius is reached for the first time, preferably as from which the temperature of 95° Celsius is reached for the first time, and preferably below 110° Celsius, preferably below 100° Celsius,
wherein the medical instrument (1110) is moreover adapted to measure and / or to calculate an energy input until the temperature of 85° Celsius is reached, preferably 95° Celsius, and preferably below 110° Celsius, preferably below 100° Celsius,
wherein the calculating unit (1144) is provided and adapted to calculate a parameter SP which links the above-mentioned results of the duration, the mean temperature and the energy input, and
wherein the medical instrument (1110), in particular the calculating unit (1144,) is adapted to switch off the medical instrument (1110), at and / or as from a predetermined value SP, preferably at a predetermined value SP between 2 and 3, in particular at and / or as from a value SP of 2.5, wherein the parameter SP is the result from the duration for which the temperature of the tissue is above 85° Celsius, preferably above 95° Celsius, and preferably below 110° Celsius, preferably below 100° Celsius, multiplied by the mean temperature as from the first time when the temperature of 85° Celsius is reached, preferably 95° Celsius, and preferably below 110° Celsius, preferably below 100° Celsius, divided by the energy input until the temperature of 85° Celsius, preferably 95° Celsius, is reached.

12. The medical instrument (1110) according to claim 11, **characterized in that** the medical instrument (1110) comprises at least one instrument branch (1, 101, 201, 301, 401, 501, 601, 701, 801, 901), which forms at least one energizable electrode (502, 602, 702, 802, 902, 1134, 1136) for sealing and / or cutting of tissue, or is arranged in or on the at least one energizable electrode (2, 102, 202, 302, 402, 702) for sealing and / or cutting of tissue, wherein the energization of the electrode (2, 102, 202, 302, 402, 502, 602, 702, 802, 902, 1134, 1136) is controllable and / or adjustable by the calculating unit (1144).

**Revendications**

1. Support de stockage lisible par ordinateur d'un instrument médical avec un dispositif de mesure de température pour mesurer la température d'un tissu, de préférence d'un instrument médical selon la revendication 11, sur lequel un procédé de détermination d'un temps d'arrêt de l'instrument médical présente les étapes suivantes :

   - la mesure d'une durée temporelle lors de laquelle une température d'un tissu est supérieure à 85° Celsius, de préférence supérieure à 95° Celsius et préférentiellement inférieure à 110° Celsius, de préférence inférieure à 100° Celsius,
   - le calcul, de préférence en ligne, d'une température moyenne à partir du moment où la température de 85° Celsius est atteinte pour la première fois, de préférence de 95° Celsius et préférentiellement lorsqu'elle est inférieure à 110° Celsius, de préférence inférieure à 100° Celsius,
   - la mesure et/ou le calcul d'un apport d'énergie jusqu'à ce que soit atteinte la température de 85° Celsius, de préférence la température de 95° Celsius et préférentiellement lorsqu'elle est inférieure à 110° Celsius, de préférence inférieure à 100° Celsius,
   - le calcul d'un paramètre SP qui relie les résultats précédents et en cas de valeur prédéterminée du paramètre SP, de préférence en cas de valeur prédéterminée SP entre 2 et 3, en particulier en cas de valeur prédéterminée SP de 2,5, l'arrêt de l'instrument médical est enregistré et amène celui-ci à exécuter le procédé lors de l'exécution par un ordinateur,

   dans lequel le paramètre SP est le résultat de la durée lors de laquelle la température du tissu est supérieure à 85° Celsius, de préférence supérieure à 95° Celsius et préférentiellement inférieure à 110° Celsius, de préférence inférieure à 100° Celsius, multipliée par la température moyenne à partir du moment où la température de 85° Celsius est atteinte pour la première fois, de préférence 95° Celsius et préférentiellement lorsqu'elle est inférieure à 110° Celsius, de préférence lorsqu'elle est inférieure à 100° Celsius, divisée par l'apport d'énergie jusqu'à ce que la température de 85° Celsius, de préférence 95° Celsius soit atteinte.

2. Support de stockage selon la revendication 1, **caractérisé en ce que** le procédé présente de plus les étapes suivantes

   - l'émission de lumière avec un spectre d'excitation dans un tissu au moyen d'au moins un éclairage (10, 1110),
   - la réception de la luminance de réflexion de la lumière, avec un spectre de réflexion, à partir du tissu par au moins un détecteur (12, 1112),
   - la conversion du spectre de luminance de réflexion au moyen du détecteur (12, 1112) en un signal de détecteur,
   - l'envoi du signal de détecteur à une unité de calcul (1144),
   - le calcul du spectre de luminance de réflexion à partir du signal de détecteur au moyen de l'unité de calcul (1144),
   - le calcul d'un spectre d'absorption du tissu en comparant le spectre d'excitation au spectre de luminance de réflexion au moyen de l'unité de calcul (1144),
   - le calcul d'au moins un maximum d'absorption à partir du spectre d'absorption au moyen de l'unité de calcul (1144) et
   - le calcul d'une température dans le tissu en comparant le maximum d'absorption à au moins une référence au moyen de l'unité de calcul (1144).

3. Support de stockage selon l'une quelconque des revendications précédentes, **caractérisé en ce que** le procédé présente de plus l'étape suivante

   - l'enregistrement d'au moins une référence sous la forme d'un maximum d'absorption à une température déterminée dans l'unité de calcul, de préférence un support de stockage dans l'unité de calcul, de préférence pour de l'eau et/ou de la graisse et/ou du collagène.

4. Support de stockage selon l'une quelconque des revendications précédentes, **caractérisé en ce que** le procédé présente de plus l'étape suivante

   - l'application de l'éclairage et du détecteur (12, 1120) sur le tissu.

5. Support de stockage selon l'une quelconque des revendications précédentes, **caractérisé en ce que** le procédé présente de plus l'étape suivante

   - la commande et/ou la régulation et/ou l'arrêt d'un dispositif, de préférence d'un instrument médical, au moyen de l'unité de calcul sur la base de la température calculée et/ou de l'impédance de tissu.

6. Support de stockage selon la revendication 5, **caractérisé en ce que** la commande et/ou la régulation et/ou l'arrêt a lieu lorsqu'une température prédéterminée est atteinte, de préférence à une température supérieure à 85° Celsius, de préférence supérieure à 95° Celsius et de préférence inférieure à 100° Celsius.

7. Support de stockage selon la revendication 5 ou 6, **caractérisé en ce que** la commande et/ou la régulation et/ou l'arrêt d'un instrument médical est effectué en ligne, de préférence en temps réel.

8. Support de stockage selon l'une quelconque des revendications précédentes, **caractérisé en ce que** le procédé de mesure de la température est réalisé pendant un processus de scellement.

9. Support de stockage selon l'une quelconque des revendications 2 à 8, **caractérisé en ce que** les détecteurs sont prévus et adaptés afin de mesurer la luminance de réflexion, de préférence les spectres de luminance de réflexion, dans la plage NIR allant de 1000 nm à 1700 nm, le plus préférentiellement dans la plage allant de 1400 nm à 1600 nm.

10. Support de stockage selon l'une quelconque des revendications précédentes, **caractérisé en ce que** l'éclairage et le détecteur sont espacés.

11. Instrument médical (1110) pour le scellement et/ou la découpe de tissu,

> avec un dispositif de mesure de température qui est prévu et adapté afin de mesurer une température d'un tissu et
> avec une unité de calcul (1144),
> dans lequel l'instrument médical (1110), en particulier le dispositif de mesure de température est prévu et adapté afin de mesurer une durée temporelle lors de laquelle la température du tissu est supérieure à 85° Celsius, de préférence supérieure à 95° Celsius et préférentiellement inférieure à 110° Celsius, de préférence inférieure à 100° Celsius,
> dans lequel l'unité de calcul (1144) est prévue et adaptée afin de calculer, de préférence en ligne, une température moyenne à partir du moment où la température de 85° Celsius est atteinte pour la première fois, de préférence à partir du moment où la température de 95° Celsius est atteinte pour la première fois et préférentiellement lorsqu'elle est inférieure à 110° Celsius, de préférence inférieure à 100° Celsius,
> dans lequel l'instrument médical (1110) est de plus adapté afin de mesurer et/ou de calculer un apport d'énergie jusqu'à ce que soit atteinte la température de 85° Celsius, de préférence la température de 95° Celsius et préférentiellement lorsqu'elle inférieure à 110° Celsius, de préférence inférieure à 100° Celsius,
> dans lequel l'unité de calcul (1144) est prévue et adaptée afin de calculer un paramètre SP qui relie les résultats précédents de la durée temporelle, de la température moyenne et de l'apport d'énergie et
> dans lequel l'instrument médical (1110), en particulier l'unité de calcul (1144), est adapté afin d'arrêter l'instrument médical (1110) en cas de et/ou à partir d'une valeur prédéterminée SP, de préférence en cas de valeur prédéterminée SP entre 2 et 3, en particulier en cas de et/ou à partir d'une valeur SP de 2,5,
> dans lequel le paramètre SP est le résultat de la durée lors de laquelle la température du tissu est supérieure à 85° Celsius, de préférence supérieure à 95° Celsius et préférentiellement inférieure à 110° Celsius, de préférence inférieure à 100° Celsius, multipliée par la température moyenne à partir du moment où la température de 85° Celsius est atteinte pour la première fois, de préférence 95° Celsius et préférentiellement lorsqu'elle est inférieure à 110° Celsius, de préférence lorsqu'elle est inférieure à 100° Celsius, divisée par l'apport d'énergie jusqu'à ce que la température de 85° Celsius, de préférence 95° Celsius soit atteinte.

12. Instrument médical (1110) selon la revendication 11, **caractérisé en ce que** l'instrument médical (1110) présente au moins une branche d'instrument (1, 101, 201, 301, 401, 501, 601, 701, 801, 901) qui forme au moins une électrode (502, 602, 702, 802, 902,1134,1136) qui peut être alimentée en courant pour le scellement et/ou la découpe de tissu ou dans ou au niveau de l'au moins une électrode (2, 102, 202, 302, 402, 702) qui peut être alimentée en courant pour le scellement et/ou la découpe de tissu, dans lequel l'alimentation en courant de l'électrode (2, 102, 202, 302, 402, 502, 602, 702, 802, 902, 1134,1136) peut être commandée et/ou régulée par l'unité de calcul (1144).

Fig. 1

Fig. 2

Fig. 3

Fig. 4

Fig. 5

Fig. 6

Fig. 8

Fig. 7

Fig. 10

Fig. 9

Fig. 12

Fig. 11

Fig. 14

Fig. 13

Fig. 16

Fig. 15

Fig. 18

Fig. 17

Fig. 20

Fig. 19

Fig. 22

Fig. 21

Fig. 23

1028

Fig. 24

1016

1014

Fig. 25

1100

1146 1144

1142

1140

1136 1138 1114 1116

1118 1130 1112

1134 1132 1110

95°C

t1    t2    t

Fig. 26

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3011924 A1
- US 2012245576 A1
- US 2016166309 A1
- US 2018345029 A1
- US 2017020597 A1